# EUROPÄISCHE PATENTSCHRIFT

(11) **EP 3 313 480 B1**
(45) Veröffentlichungstag und Bekanntmachung des Hinweises auf die Patenterteilung: **05.05.2021**
(21) Anmeldenummer: 16734167.6
(22) Anmeldetag: 16.06.2016
(51) Int. Cl.: A61M 5/20, A61M 5/315, A61M 5/46, A61M 5/48, A61M 5/31, A61M 5/32

(54) **VERBESSERTE ANTRIEBSEINHEIT UND INJEKTIONSVORRICHTUNG**
IMPROVED DRIVE UNIT AND INJECTION DEVICE
UNITÉ D'ENTRAÎNEMENT ET DISPOSITIF D'INJECTION AMÉLIORÉS

(30) Priorität: 23.06.2015 CH 9042015; 21.04.2016 CH 5312016
(43) Veröffentlichungstag der Anmeldung: 02.05.2018
(73) Patentinhaber: Ypsomed AG, 3401 Burgdorf (CH)
(72) Erfinder: SCHRUL, Christian, 3400 Burgdorf (CH); TSCHIRREN, Markus, 3400 Burgdorf (CH); SCHENKER, Susanne, 4912 Aarwangen (CH); HOSTETTLER, Patrick, 3415 Hasle (CH); STREIT, Ursina, 3322 Schönbühl (CH)
(74) Vertreter: Kleiner, Stefan
(86) Internationale Anmeldenummer: PCT/CH2016/000093
(87) Internationale Veröffentlichungsnummer: WO 2016/205963

(56) Entgegenhaltungen:
- WO-A1-2007/115424
- WO-A1-2014/111370
- WO-A1-2014/154491
- WO-A1-2014/170267
- WO-A1-2014/191189

## Beschreibung

Die vorliegende Patentanmeldung bezieht ihr Prioritätsdatum von der Patentanmeldung 00904/15 (CH), welche am 23.06.2015 beim Eidgenössischen Institut für Geistiges Eigentum in der Schweiz eingereicht worden ist.

Die Erfindung betrifft ein Federpaket für eine Injektionsvomchtung, eine entsprechende Injektionsvorrichtungen sowie ein Verfahren zur Montage dieser Injektionsvorrichtungen. Im Besonderen betrifft die Erfindung eine verbesserte Antriebseinheit für eine Injektionsvorrichtung und ein Verfahren zur Montage dieser Antriebseinheit in der Injektionsvorrichtung.

Der Begriff "Medikament" oder Produkt umfasst hier jede fliessfähige medizinische Formulierung, welche geeignet ist zur kontrollierten Verabreichung durch ein Mittel, wie z. B. eine Kanüle oder Hohlnadel, hindurch, beispielsweise umfassend eine Flüssigkeit, eine Lösung, ein Gel oder eine feine Suspension, welche(s) einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament oder Produkt umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vakzine, DNS oder RNS oder Oligonukleotide, Antikörper oder Teile von Antikörpern sowie geeignete Basis-, Hilfs- und Trägerstoffe.

Aus der WO2014111370A1 sind Energiespeichereinheiten für Verabreichungsgeräte entsprechend der Präambel von Anspruch 1 bekannt.

Aus der WO2014/170267 sind Injektionsgeräte mit einem kompakten Energiespeicher bekannt. Der Energiespeicher umfasst mindestens zwei miteinander verbundene Spiralfedern, welche genügend Energie gespeichert haben können, i.e. vorgespannt sind, um einen ganzen Medikamentenbehälter zu entleeren. Die Schrift offenbart keine Details zur Art und Weise wie die Spiralfedern vorgespannt werden können.

AusderWO2014/154491 ist eine Energiespeichereinheit für ein Verabreichungsgerät bekannt. Diese Energiespeichereinheit umfasst eine vorgespannte Druckfeder, wobei die Einheit über eine Transportverriegelung verfügt, welche eine ungewollte Aktivierung der Energiespeichereinheit verhindern soll. Zur Verabreichung von zähflüssigen Medikamenten müssen Druckfedem gross und lang dimensioniert werden, so dass auch die Verabreichungsgeräte grösser dimensioniert werden müssen. Um eine regelmässige Verabreichung von Medikament sodann erzielen zu können, müssen Druckfedem stark vorgespannt werden, was bei Verwendung von Fertigspritzen aus Glas als Medikamentenbehälter das Glasbruchrisiko bei Auslösung der Verabreichungsvorrichtung führen erhöht.

Es wäre deshalb wünschenswert Energiespeichereinheiten zur Hand zu haben, welche mit gespeicherter Energie einfach zu transportieren wären und geometrisch auch in bekannte Verabreichungsvorrichtung einpassbar wären.

Es ist daher eine Aufgabe der Erfindung, einen verbesserten Energiespeicher für eine Verabreichungsvorrichtung anzugeben, welcher das Ausschütten von hochviskosen Medikamenten ermöglicht und dabei kosteneffizient herstellbar ist wie auch eine kosteffiziente Herstellung der Verabreichungsvorrichtung erlaubt.

Die Aufgabe wird mit einem Federpaket, einer Verabreichungsvorrichtung, sowie einem Verfahren zur Montage eines Federpaketes nach den Ansprüchen 1,10 respektive 11 gelöst. Vorteilhafte Weiterentwicklungen ergeben sich aus den abhängigen Ansprüchen, der Beschreibung und den Figuren.

In einem Aspekt betrifft die Erfindung ein Federpaket für eine Verabreichungsvorrichtung umfassend eine Feder, welche als Trieb- oder Spiralfeder ausgebildet ist und einen Federschaft mit einem fest angebrachten Flansch, wobei das innere Ende der Feder zumindest drehfest am Schaft angebracht ist. Weiter umfasst das Federpaket eine Federhülse, welche den Mantelbereich der Feder zumindest teilweise umgibt, wobei das äussere Ende der Feder drehfest mit der Federhülse verbunden ist, und einen scheibenartigen Federhülsendeckel, welcher axial fest an der Federhülse oder dem Federschaft angebracht werden kann, wobei der Tellerdurchmesser des Federhülsendeckel gleich oder kleiner ist als derjenige der Federhülse. Der Federschaft definiert eine Achse, weist ein proximales und ein distales Ende auf, wobei sich Flansch und Federhülsendeckel radial von der Achse weg erstrecken und der Flansch in der Nähe des distalen Endes des Federschaftes angeordnet ist. Wobei weiter an der Peripherie des Flansches mindestens ein radialer Anschlag angeordnet ist, und an der Federhülse komplementär zum mindestens einen radialen Anschlag mindestens ein Blockiermittel angeordnet ist, so dass bei einem Eingriff von Blockiermittel und radialem Anschlag die Federhülse und der Federschaft relativ zueinander und lösbar verdrehgesichert werden.

In einem weiteren Aspekt betrifft die Erfindung ein Federpaket, bei welchem die axiale Positionierung der Feder durch die feste Position des Flansches, sowie eine variable axiale Fixierung des Federhülsendeckels definiert wird und dadurch Federn mit unterschiedlicher axialer Ausdehnung im Federpaket eingesetzt werden können.

Wobei in einer Fortbildung der Federhülsendeckel in die Federhülse hineinverschoben werden kann, wobei der Federhülsendeckel an seinem Umfang mindestens ein Schnappelement aufweist, und die Federhülse dazu komplementär mindestens eine Aufnahme für das mindestens eine Schnappelement aufweist, um den Federhülsendeckel an der Federhülse zu befestigen.

Wobei in einer weiteren Fortbildung die Federhülse an mehreren axialen Positionen mindestens eine Aufnahme für das mindestens eine Schnappelement des Federhülsendeckels umfasst, so dass der Federhülsendeckel in verschiedenen axialen Positionen an der Federhülse befestigt werden kann.

In einem Aspekt kann die Feder aus spiralig aufgerolltem Bandmaterial bestehen, bevorzugt aus metallischem Bandmaterial, besonders bevorzugt aus bandförmigem Stahl.

In einem weiteren Aspekt umfasst die Erfindung ein Federpaket wie beschrieben, wobei die Federhülse koaxial zum Federschaft angeordnet ist, dadurch gekennzeichnet, dass an der Federhülse zumindest ein flexibler Arm angeordnet ist, welcher sich in Umfangsrichtung der Federhülse erstreckt, welcher mit einem Ende fest an der Federhülse angebracht ist und am anderen Ende in radiale Richtung ausgelenkt werden kann und wobei am freien Ende das Blockiermittel, insbesondere in der Form eines Zahnes angeordnet ist, welcher durch Auslenkung des zumindest einen flexiblen Armes des flexiblen Armes in Eingriff mit dem radialen Anschlag gebracht werden kann oder aus dem Eingriff gelöst werden kann, so dass der flexible Arm zusammen mit dem Blockiermittel einen Sperrschnapper bilden kann. Weiter kann am freien Ende des Armes axial in distale Richtung zum Blockiermittel versetzt ein Steuerarm angeordnet sein, und wobei bei radialer Auslenkung des Steuerarmes auch das Blockiermittel entsprechend radial bewegt wird und umgekehrt.

In einem weiteren Aspekt kann der Federschaft eine axial ausgebildete Halterippe aufweisen, in welcher das als Haltelasche ausgebildete innere Ende der Feder drehfest verankert werden kann, und wobei die Federhülse eine axial orientierte Haltestruktur aufweist, an welcher das als Haltelasche ausgebildete äussere Ende der Feder drehfest verankert werden kann.

Die Feder kann nach einer Ausgestaltung durch relative Drehung der Federhülse zum Federschaft vorgespannt werden, wobei diese Vorspannung durch einen Eingriff von radialen Anschlag und Blockiermittel gehalten werden kann, wobei die Vorspannung einem Drehmoment von 1 bis 100 Nmm entsprechen kann, bevorzugt einem Drehmoment von 30 bis 80 Nmm entsprechen kann und besonders bevorzugt einem Drehmoment von 60 bis 70 Nmm entsprechen kann.

In einem Aspekt bezieht sich die Erfindung auf eine Verabreichungsvorrichtung zur Verabreichung eines fluiden Produktes, die Verabreichungsvorrichtung umfassend eine Längsachse und ein Gehäuse mit einem Mechanikhalter welcher fest mit dem Gehäuse verbunden ist. Weiter umfasst die Verabreichungsvorrichtung eine Auslösevorrichtung sowie einen Produktbehälter, insbesondere in der Form einer vorbefüllten Spritze oder Karpule, welcher zumindest axial fest in einem Teil des Gehäuses angeordnet ist, wobei im Produktbehälter ein axial verschiebbarer Stopfen angeordnet ist, durch dessen Verschiebung in distale Richtung Produkt aus dem Produktbehälter ausgeschüttet werden kann. Noch weiter umfasst die Verabreichungsvorrichtung ein erfindungsgemässes Federpaket, in welchem Energie für das automatische Ausschütten von Produkt gespeichert ist, wobei das Federpaket operativ mit der Auslösevorrichtung verbunden ist, und wobei die Federhülse drehfest mit dem Gehäuse verbunden ist,eine koaxial zur Längsachse angeordnete Gewindestange, welche drehfest mit dem Federschaft verbunden ist, eine koaxial zur Längsachse angeordnete Kolbenstange, welche axial verschiebbar und drehfest im Mechanikhalter geführt wird, wobei die Kolbenstange mit dem Stopfen des Produktbehälters so wechselwirken kann, dass bei axialen Bewegung der Kolbenstange in distale Richtung auch der Stopfen in distale Richtung verschoben werden kann, wobei die Kolbenstange hülsenartig aufgebaut ist und über Gewindeelemente auf einer inneren Oberfläche der Kolbenstange in Gewindeeingriffe mit der Gewindestange ist, so dass eine Rotation der Gewindestange, eine axiale Verschiebung der Kolbenstange zur Folge hat.

Weiter betrifft die Erfindung ein Verfahren zur Montage eines Federpaketes mit einer vorgespannten Trieb-oder Spiralfeder in eine Verabreichungsvorrichtung zur Verabreichung eines fluiden Produktes, insbesondere eine längliche Injektionsvorrichtung, mit einem Gehäuse und einem gehäusefesten Mechanikhalter umfassend zumindest folgende Schritte:
a) Das Federpaket wird axial auf eine vormontierte Antriebseinheit der Verabreichungsvorrichtung, welche den Mechanikhalter umfasst, geschoben, wodurch ein drehbares in der Antriebseinheit vorhandenes Antriebsglied mit einem Federschaft des Federpaketes verdrehsicher verbunden wird, und wodurch sich ein fest am Mechanikhalter angeordnetes Freigabeelement vor einen Steuerarm des Federpaketes schiebt
b) Ein Gehäuseteil oder Abschlussteil der Verabreichungsvorrichtung wird in distale Richtung axial über das Federpaket geschoben, wodurch die äussere Mantelfläche des Federpaketes verdrehgesichert mit dem Gehäuseteil oder Abschlussteilverbunden wird.
c) das Gehäuseteil oder Abschlussteil wird relativ zu Federpaket und vormontierter Antriebseinheit verdreht, wodurch die Führungselemente des Mechanikhalters in Führungen des Gehäuseteils oder Abschlussteil geführt werden, wobei die Führung entlang des Umfangs des Gehäuseteils oder Abschlussteils verläuft, wobei durch die Verdrehung das Freigabeelement den Steuerarm in radiale Richtung nach aussen bewegt und somit ein im Federpaket vorhandenes Drehmoment freigibt, wodurch eine weitere relative Rotation zwischen Gehäuse- oder Abschlussteil und dem Mechanikhalter evoziert bis ein radialer Block des Federpaketes in Eingriff mit einem radialen Anschlag der Mechanikhalter gerät und so eine kraftschlüssige Verbindung zwischen Federpaket und Mechanikhalter erzeugt wird.

In einem Aspekt betrifft die Erfindung eine Verabreichungsvorrichtung, welche nach dem vorhergehenden Absatz beschriebenen Verfahren montiert wurde, wobei die Trieb- oder Spiralfeder so gewickelt ist, dass das Drehmoment, welches auf die äussere Mantelfläche des Federpaketes nach der Freigabe des Drehmomentes wirkt, verglichen mit dem entlang des Umfangs verlaufenden Teiles der Führung am Gehäuseteil in die entgegensetzte tangentiale Richtung weist.

In einem Aspekt betrifft die Erfindung eine Verabreichungsvorrichtung, welche nach dem vorhergehenden Absatz beschriebenen Verfahren montiert wurde, wobei es sich bei der Verabreichungsvorrichtung um einen Autoinjektor oder einen Injektionspen mit automatischer Verabreichung handelt,

In einem Aspekt betrifft die Erfindung eine Verabreichungsvorrichtung, welche nach dem vorhergehenden Absatz beschriebenen Verfahren montiert wurde, wobei es sich bei der Verabreichungsvorrichtung um ein ,patch'-Gerät handelt.

Der Begriff ,distal' bezieht sich auf das geometrische Ende Verabreichungsvorrichtung an welchem das Medikament bei Verabreichung austritt. Der Begriff proximal bezieht sich somit auf das entgegengesetzte Ende. Bei in einem penartigen Injektionsgerät entspricht das distale Ende demjenigen mit der Injektionsnadelspitze.

Der Begriff, radialer Anschlag' bezieht sich auf einen Anschlag, welcher relative Rotation zwischen zwei Teilen unterbinden kann. Ein radialer Anschlag besteht beispielsweise aus einer flächigen Struktur, deren Flächennormal tangential zu einem durch die Rotation virtuell generierten Kreis steht.

Es wird ein Federpaket für eine Verabreichungsvorrichtung offenbart, welches zumindest aus einem Federschaft, einer Feder, insbesondere einer Spiral- oder Uhrenfeder, und einer Federhülse besteht. Die Feder kann mechanisch gespannt werden und in der Folge ein Drehmoment auf den Federschaft oder die Federhülse ausüben. Die Feder ist mit einem Ende mit dem Schaft verbunden und mit dem anderen Ende mit der Hülse.

In einer vorteilhaften Anordnung ist die Feder einer Spiralfeder, weiche um den Federschaft herum gewickelt ist und deren inneres Ende mit dem Federschaft verbunden ist Bei dieser Anordnung ist das äussere Ende der Feder mit der Federhülse verbunden, wobei die Federhülse für die Feder einen Mantel bildet. In dieser Anordnung kann die Feder gespannt werden, in dem die Federhülse relativ zu Federschaft verdreht wird.

Die Federhülse wird relativ zum Federschaft gegen Verdrehung gesichert. Hierbei kann an der Federhülse ein Blockierelement fest angeordnet sein und am Federschaft kann ein komplementär dazu ausgebildetes Element fest angeordnet sein - welche bei Zusammenwirken als Verdrehsicherung funktionieren können. Durch Bewegen eines der Elemente kann sodann die Verdrehsicherung gelöst werden. Das Blockierelement ist als Sperrschnapper ausgebildet, welcher in einen radialen Anschlag am Federschaft eingreifen kann. Dabei kann der Sperrschnapper als flexibler Arm an der Federhülse angeordnet sein oder Teil der Hülse sein. An einem freien Ende des Arms kann so dann zum Beispiel ein Zahn angeordnet sein, welcher in Eingriff mit dem radialen Anschlag gebracht werden kann. Der Eingriff zwischen Anschlag und Zahn kann durch eine Bewegung des freien Endes des Armes, insbesondere eine Bewegung in radiale Richtung nach aussen gelöst werden.

Das Federpaket enhält eine vorgespannte Feder, wobei die Federspannung von der oben beschriebenen Vedrehsicherungen gehalten wird. In einer vorteilhaften Ergänzung umfasst das Federpaket ein Deckel- und ein Bodenelement. In einer weiteren Fortentwicklung ist das Bodenelement als Flansch fest auf dem Federschaft angeordnet und das Deckelement ist als separates Element ausgebildet, welches in variablem Abstand zum Bodenelement am Federschaft und/oder an der Federhülse fixiert werden kann, dass die Feder vor äusseren mechanischen Einflüssen geschützt werden kann. In der Folge kann das Federpaket bei vorgespannter Feder transportiert werden. Die Feder kann durch das Boden- und/oder Deckelement auch in axialer Richtung gestützt und/oder geführt werden. In einer alternativen Ausgestaltung kann das Federpaket auch zwei, drei oder mehr vorgespannte Federn enthalten, vorzugsweise mit einem Deckelelement für jede Feder.

In einer vorteilhaften Anordnung besteht die Feder aus spiralförmigem Bandmaterial, wobei die Federparameter wie die Federkonstante unter anderen durch die Breite des Bandes bestimmt werden. Die oben beschiebenen Anordnungen erlauben es vorteilhaft verschieden steife Federn bei identischem Federschaft (auch mit Flansch), identischer Federhülse und identischem Deckelement zu verbauen. Das verwendete Bandmaterial kann Metall, insbesondere Stahl, oder Kunststoff sein.

In einem weiteren Aspekt der Erfindung wird eine Verabreichungvorrichtung offenbart, in welche ein Federpaket nach einer der obigen Anordnungen eingebaut werden kann, wobei das Federpaket als Antriebsmittel dienen kann. Bei der Art der Verabreichungsvorrichtung kann es sich um eine penartige Injektionsvorrichtung mit einem Medikamentenspeicher handeln, insbesondere einen Autoinjektor oder einen Autopen. Dabei kann die penartige Injektionsvorrichtung für das Verabreichen von genau einer Dosis respektive von mehreren Dosen ausgelegt sein. Auch kann die Injektionsvorrichtung ein Mittel umfassen, mit welchem die Grösse der Dosis gezielt gewählt werden kann. Das Federpaket ist in vorteilhaften Formen vorgespannt, so dass der Medikamentenspeicher teilweise oder vollständig durch die in der Feder gespeicherte Energie verbreicht werden kann. In weiteren interessanten Ausgestaltung ist im Federpaket mehr Energie gespeichert als zum Verabreichen eines Medikamentenspeichers benötigt wird. Dadurch kann das Federpaket insbesondere auch in wiederverwendbaren Injektionsvorrichtungen eingesetzt werden. Alternativ kann das Federpaket auch so ausgestaltet sein, dass die Feder durch eine benutzende Person gespannt werden kann, zum Beispiel beim Wechseln des Medikamentenspeichers oder beim Einstellen einer Dosis.

In einem alternativen Aspekt wird eine Verabreichungsvorrichtung offenbart, in welche ein Federpaket nach einer der obigen Anordnungen eingebaut werden kann, wobei auch hier das Federpaket als Antriebsmittel dienen kann. Bei diesem alternativen Aspekt kann es sich bei der Art der Verabreichungsvorrichtung um eine Injektions-oder Infusionsvorrichtung handeln, welche mittels insbesondere einer Klebeverbindung direkt auf die Haut eines Patienten aufgebracht werden kann. Solche Vorrichtungen sind dem Fachmann als ,patch injector oder,patch pump' bekannt. In einer weiteren Ausgestaltung des Aspektes kann das Federpaket nicht nur als Antriebsmittel für den Verabreichungsprozess dienen, sondern auch oder alternativ als Antriebsmittel zum Einstechen der Verabreichungskanüle durch welche ein Produkt verabreicht wird.

In einem weiteren Aspekt der Erfindung wird ein Verfahren zur Montage eines Federpaketes in eine Verabreichungsvorrichtung offenbart. Dabei kann es sich bei der Verabreichungsvorrichtung um eine penartige Injektionsvorrichtung handeln. Alternativ kann es sich auch um die obengenannten ,patch'-Geräte handeln.

In einer vorteilhaften Ausgestaltung des Verfahrens zur Montage des Federpaketes in die Verabreichungsvorrichtung, handelt es sich bei der Verabreichungsvorrichtung um eine penartige Injektionsvorrichtung, insbesondere ein Autoinjektor oder einen Autopen. Bei der Montage des Federpaketes wird wie folgt beschrieben das Federpaket auf einer vormontierten Antriebseinheit angeordnet. Die vormontierte Antriebseinheit besteht dabei zumindest aus einer Federaufnahme, insbesondere einem Mechanikhalter, und ein beweglich, insbesondere drehbar, in der Federaufnahme oder Mechanikhalter angeordnetes Antriebsglied. In einer Ausführungsform mit einem Mechanikhalter kann das Antriebsglied eine Gewindestange sein, welche an ihrem proximalen Ende eine Längsführung aufweist.

Es wird in einem ersten Montageschritt, das Federpaket auf das proximale Ende der vormontierten Antriebseinheit geschoben. Dabei wird die beschriebene Gewindestange mit dem Federschaft in eine verdrehgesicherte Verbindung gebracht, so dass eine Rotationsbewegung des Schaftes auf die Gewindestange übertragen wird. Im Federpaket sind in diesem Zustand Blockiermittel der Federhülse und das dazu komplementäre Element in Eingriff, so dass die Feder des Federpaketes vorgespannt sein kann. Auf dem Mechanikhalter ist welter ein Freigabeelement angeordnet, welches beim Aufschieben des Federpaketes relativ zum Blockiermittel in eine Auslöseposition gelangt. Ausgehend von dieser Auslöseposition ist eine weitere relative Bewegung zwischen Freigabeelement und Blockiermittel möglich, durch welche der Eingriff zwischen Blockiermittel und dem komplementären Element gelöst wird. In dieser sogenannten ausgelösten Position ist nun eine relative Drehung zwischen Federschaft und Federhülse möglich, das heisst, dass in diesem Zustand Drehmoment von der Feder nach ausserhalb des Federpaketes geleitet werden kann.

In einem zweiten Montageschritt wird ein Gehäuseteil der Injektionsvorrichtung in distale Richtung über das Federpaket und zumindest auch teilweise über die vormontierte Antriebseinheit geschoben. Dabei wird die Federhülse zum Gehäuseteil verdrehgesichert. Weiter wird eine Verbindung zwischen Mechanikhalter und einem Gehäuseteil hergestellt, welche in folgender Konsequenz, Gehäuse und Mechanikhalter der Injektionsvorrichtung fest miteinander verbindet.

In einer vorteilhaften Ausgestaltung der Verbindung zwischen Mechanikhalter und Gehäuseteil wird die Verbindung durch einen Bajonettverschluss hergestellt. Diese Art der Verbindung hat den Vorteil das bei der Herstellung der festen Verbindung auch das Freigabeelement des Mechanikhalters von der Auslöseposition in die ausgelöste Position bewegt werden kann. Hierzu können auf der Peripherie des Mechanikhalters Bajonettnocken angeordnet sein, welche in Eingriff mit Bajonettnuten auf einer Innenfläche des Gehäuseteils gelangen können. Die Bajonettnuten haben dabei mindestens einen Teil, welcher axial verläuft und mindestens einen weiteren Anteil, welcher in Umfangsrichtung verläuft. Im zweiten Montageschritt gelangen die Bajonettnuten in Eingriff mit dem axialverlaufenden Teil der Bajonettnuten, so dass der Gehäuseteil über den Mechanikhalter geschoben werden kann. Am Ende dieser Bewegung ist die Federhülse verdrehgesichert mit dem Gehäuseteil verbunden.

Im Folgenden dritten Montageschritt wird der Mechanihalter relativ zum Gehäuseteil verdreht, wobei die Bewegung durch den Anteil der Bajonettnut geführt wird, welcher in Umfangsrichtung verläuft und wobei das Bajonett geschlossen wird. Gleichzeitig, da die Federhülse nun verdrehgesichert zum Gehäuseteil ist, findet eine relative Bewegung zwischen Mechanikhalter und Federhülse statt, was zur Folge hat, dass das Freigabelement von der Auslöseposition in die ausgelöste Position bewegt wird.

In einer alternativen Ausführung kann die Federaufnahme auch über eine Schnappververbingung mit einem als Abschlussteil ausgebildeten Gehäuseteil verbunden werden. Wobei die Schnappung den ersten Teil des Bajonettverschlusses ersetzt und das Abschlussteil nach der Verschnappung verdrehbar in der Federaufnahme gelagert ist, so dass der obige, dritte Montageschritt gleich wie beim Bajonettverschluss vollzogen werden kann.

Alternativ kann die Verbindung zwischen Federaufnahme und Abschlussteil auch durch Gewindeteile mitgeformt werden.

### Figuren

- Figur 1: Ansicht einer erfindungsgemässen Injektionsvorrichtung mit einem erfindungsgemässen Federpaket.
- Figuren 2a-2b: Schnittansicht, resp. explodierte Ansicht der Injektionsvorrichtung aus Figur 1.
- Figuren 3a-3c: Aussenansicht, Schnittansicht, respektive explodierte Ansicht eines erfindungsgemässen Federpaketes
- Figuren 4a-4c: Aussenansicht, Schnittansicht, respektive explodierte Ansicht eines erfindungsgemässen Federpaketes mit einer alternativen Ausführung der Spiralfeder
- Figuren 5a-5b: Ansicht eines erfindungsgemässen Federpaketes aus distaler Position, wobei Figur 5b ein Detailausschnitt B aus Figur 5a wiedergibt
- Figur 6: Ansicht der ersten Montagephase
- Figur 7a: Ansicht der zweiten Montagephase
- Figur 7b: Ansicht der Endkappe mit Bajonettnut
- Figur 8: Ansicht der dritten Montagephase
- Figuren 9a-d: Ansichten eines erfindungsgemässen Federpaketes aus distaler Position in drei verschiedenen Zuständen während der Montage, wobei der der Mechanikhalter teilweise angeschnitten gezeigt wird
- Figuren 10a-d: Ansichten des Federpaketes aus Figuren 9a-d wobei ein distaler Teil des Federpakets angeschnitten wurde um den Sperrschnapper besser zeigen zu können.
- Figur 11: Aussenansicht einer alternativen Injektionsvorrichtung
- Figuren 12a-12b: Schnitt- und Explosionsdarstellung der Injektionsvorrichtung aus Figur 11
- Figur 12c: Detaillierter Querschnitt B-B aus Figur 12b

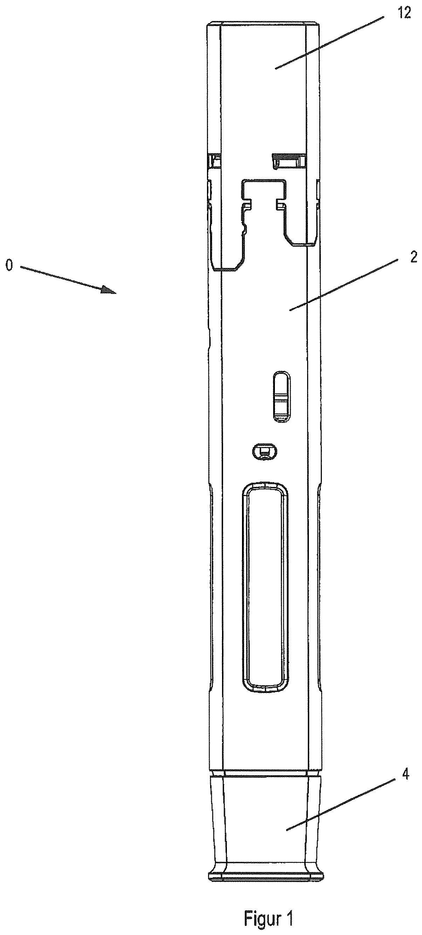

Die Figuren 1-10d zeigen ein erfindungsgemässes Federpaket mit einer entsprechenden Injektionsvorrichtung, einem Autoinjektor, sowie den verschiedenen Montageschritten des zugehörigen erfindungsgemässen Verfahrens.

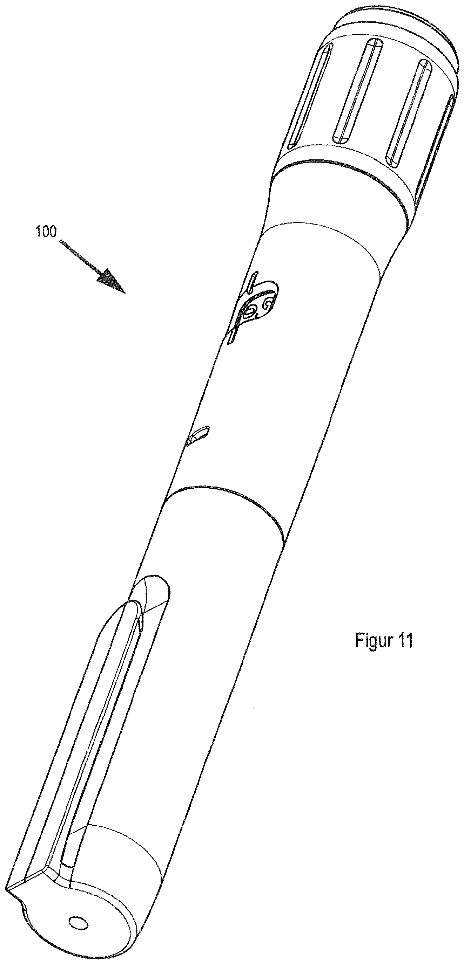

Die Figuren 11-12d zeigen danach die Anwendung erfindungsgemässer Federpakete in einer alternativen Verabreichungsvorrichtung, nämlich einem sogenannten Autopen, einem Injektionspen mit welchem eine manuell einstellbare Dosis durch gespeicherte Energie verabreicht werden kann.

Bezugnehmend auf die Figuren 1-10d werden im Folgenden eine erste Serie möglicher Ausführungen der Erfindung beschrieben, basierend auf einem Autoinjektor 0, wie er in Figur 1 dargestellt ist. Es wird an dieser Stelle die Schweizerische Patentanmeldung 00904/15 referenziert und vollständig in die vorliegende Anmeldung integriert, da sie den Autoinjektor 0 abgesehen vom Federpaket ebenfalls umfasssend beschreibt.

Es soll nun der Aufbau und die Funktion des Autoinjektors 0 anhand der Figuren 1- 2b dargelegt werden. Autoinjektor 0 weist ein hülsenförmiges, längliches Gehäuse 2 mit einer Längsachse L auf, welches an seinem proximalen Ende eine Verschlusskappe 12 aufweist, die formschlüssig mit dem Gehäuse 2 dreh- und axialfest verbunden ist und das proximale Ende des Autoinjektors bildet. Die Verschlusskappe 12 ist mit dem Gehäuse 2 verschnappt. Hierfür weist die Verschlusskappe 12 ein Rastglied 12a auf, welches in eine Ausnehmung 2a am Gehäuse 2 eingerastet ist, vorzugsweise so, dass die Verschlusskappe 12 nicht oder nicht ohne weiteres von dem Gehäuse 2 lösbar ist.

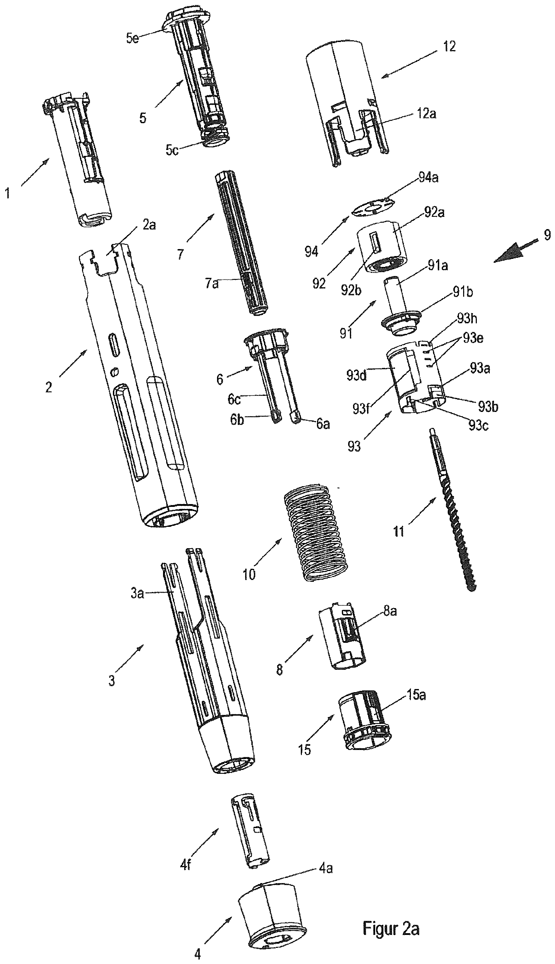

An dem distalen Ende des Autoinjektors ist in seinem Auslieferungszustand (Figuren 2a-2b) eine Abziehkappe 4 mit einem Cap Remover 4f angeordnet, die vor der Verwendung des Autoinjektors abgezogen, abgeschraubt oder abgedreht, und entfernt wird.

In dem Gehäuse 2 ist ein Produktbehälter 13 in der Gestalt einer Spritze in Bezug auf das Gehäuse 2 - abgesehen von der Montage des Autoinjektors - entlang der Längsachse L unverschlebbar aufgenommen. Der Produktbehälter 13 weist einen hülsenförmigen Spritzenkörper auf, der einen Kolben 13b, der dichtend an dem Innenumfang des Spritzenkörpers anliegt, umgibt. Der Spritzenkörper weist an seinem distalen Ende eine insbesondere unlösbar mit dem Spritzenkörper verbundene Injektionsnadel 13a auf, deren distales Ende von der Nadelspitze gebildet wird. Zwischen der Injektionsnadel 13a und dem Kolben 13b ist ein flüssiges Produkt, insbesondere Medikament, innerhalb des Spritzenkörpers angeordnet, wobei das flüssige Produkt durch Verschieben des Kolbens 13b in eine Ausschüttrichtung, d. h. in distale Richtung oder zu der Injektionsnadel 13a hin durch die hohle Injektionsnadel 13a aus dem Produktbehälter 13 ausgeschüttet wird. Der Spritzenkörper weist an seinem proximalen Ende einen sogenannten Fingerflansch auf, der radial nach außen über den Außenumfang des zylindrischen Spritzenkörpers ragt.

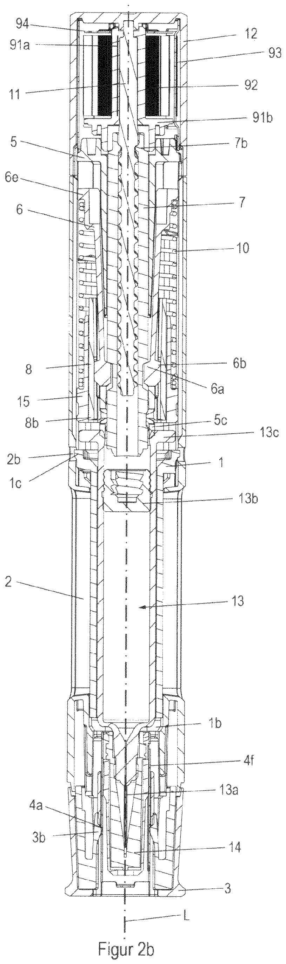

Der Produktbehälter 13 ist in einem Produktbehälterhalter, der als Spritzenhalter 1 bezeichnet wird, so aufgenommen, dass er zumindest gegen eine Bewegung entlang der Längsachse L in distale Richtung relativ zu dem Spritzenhalter 1 gesichert ist. Der Spritzenhalter 1 ist, wie am besten aus Figur 2b ersichtlich ist, mit dem Gehäuse 2 formschlüssig verbunden, insbesondere verrastet. Das Gehäuse 2 weist hierzu Ausnehmungen auf, in die Rastelemente, die hier am proximalen Ende des Spritzenhalters 1 gebildet werden, eingreifen. Der Spritzenhalter 1 weist mindestens eine nach innen ragende Schulter 1b auf, an der sich ein verjüngender Abschnitt des Produktbehälters 13, der distal des zylindrischen den Kolben 13b führenden Spritzenkörperabschnitts, angebracht ist, abstützt.

Um zu verhindern, dass der Produktbehälter 13 relativ zu dem Spritzenhalter 1 in die proximale Richtung verschiebbar ist, wird der Produktbehälter 13 an seinem proximalen Ende durch einen auf den Spritzenkörper wirkenden Halter in den Eingriff mit der Schulter 1b gedrückt. Der Halter wird von einem Haltefederabschnitt 5c eines Mechanikhalters 5 gebildet. Der Mechanikhalter 5 ist in Bezug auf das Gehäuse 2 entlang der Längsachse L insbesondere unverschiebbar und/oder drehfest angeordnet. Der hülsenförmige Mechanikhalter 5 kann mit dem Gehäuse 2 verschnappt sein. Durch den Haltefederabschnitt 5c können Längenunterschiede des Produktbehälters 13, die aufgrund von Herstellungstoleranzen entstehen können, ausgeglichen werden, wobei der feste Sitz des Produktbehälters 13 an der Schulter 1b sichergestellt ist.

Der Produktbehälter 13 ist in Bezug auf das Gehäuse 2 so angeordnet, dass die Nadelspitze distal über das distale Ende des Gehäuses 2 übersteht. Im Ausgangs- oder Auslieferungszustand des Autoinjektors, d. h., wenn die Abziehkappe 4 an dem Autoinjektor angeordnet ist, wird die Nadel 13a von einer Nadelabdeckkappe 14, die in dem gezeigten Beispiel als sogenanntes und dem Fachmann bekanntes rigid needle shield, alternativ als soft needle shield, ausgebildet ist, abgedeckt, um die Nadel 13a vor Verschmutzung zu schützen bzw. die Nadel 13a und das Medikament steril zu halten. Das rigid needle shield 14 ist an einem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers beflridet. Die Abziehkappe 4 ist mit dem Gehäuse 2 oder einer Nadelschutzhülse 3 lösbar verschnappt, wobei diese Verschnappung gelöst wird, wenn die Abziehkappe 4 von dem Gehäuse 2 oder der Nadelschutzhülse 3 entfernt wird. Die Verschnappung wird in dem gezeigten Beispiel durch einen Schnapphaken 3b der Nadelschutzhülse 3 und einer Schnappgeometrie 4a der Abziehkappe 4 gebildet. Die Abziehkappe 4 weist ferner insbesondere einen cap remover 4f auf, mit welchem das rigid needle shield 14 vom Produktbehälter 13 gelöst und zusammen mit der Abdeckkappe 4 von dem Autoinjektor entfernt wird.

Der Autoinjektor 0 weist eine Nadelschutzhülse 3 auf, die relativ zu dem Gehäuse 2 und entlang der Längsachse L um einen Betätigungshub H_{B} (nicht gezeigt) in die proximale Richtung in eine betätigte Position verschiebbar ist, um eine Produktausschüttung auszulösen. In der Ausgangsposition der Nadelschutzhülse 3, wie sie in der Figur 2b gezeigt wird, steht das distale Ende der Nadelschutzhülse 3 distal über die Nadelspitze der Nadel 13a über, so dass ein Zugriff auf die Nadelspitze zunächst verhindert wird. Durch Verschieben der Nadelschutzhülse 3 um den Betätigungshub H_{B} wird die Nadelschutzhülse 3 soweit in die proximale Richtung verschoben, dass die Nadel 13a aus dem distalen Ende der Nadelschutzhülse 3 hervortritt, insbesondere mit einer Länge hervortritt, welche der Injektionstiefe der Nadel in die Einstichstelle entspricht. Bevorzugt soll die Nadel 13a soweit über das distale Ende der Nadelschutzhülse 3 überstehen, dass eine subkutane oder intramuskuläre Injektion erfolgen kann.

Nach der erfolgten Injektion kann die Nadelschutzhülse 3 relativ zu dem Gehäuse 2 aus der betätigten Position entlang der Längsachse L um einen Nadelschutzhub H_{N} (nicht gezeigt) in die distale Richtung in eine Nadelschutzposition verschoben werden. In der Nadelschutzposition steht das distale Ende der Nadelschutzhülse 3 distal über die Nadelspitze über, so dass ein Zugriff auf die Nadelspitze verhindert und eine Verletzungsgefahr verringert wird. Die Nadelschutzhülse 3 kann, wie weiter unten beschrieben wird, gegen erneutes Zurückschieben aus der Nadelschutzposition blockiert werden.

Der Autoinjektor 0 weist ferner ein insbesondere hülsenförmiges Vortriebsglied 7 auf, welches insbesondere an seiner Innenseite einen Gewindeabschnitt insbesondere ein Gewindesegment 7b aufweist. Das Vortriebsglied 7 ist insbesondere rotativ fest in Bezug auf das Gehäuse.

Des Weiteren umfasst der Autoinjektor 0 wie bereits erwähnt ein Rotationsglied 11 insbesondere eine Gewindestange 11, dessen Drehung bewirkt, dass die Federenergie an das Vortriebsglied 7 abgegeben wird, wodurch das Vortriebsglied 7 insbesondere durch einen Gewindetrieb in distale Richtung bewegt wird. Die Gewindestange 11 ist mit einer ersten Feder in Form des Federpaketes 9 verbunden, welches die zur Produktausschüttung notwendige Energie speichert und bei Bedarf abgibt. Die Gewindestange 11 ist mit einem Ende einer ersten Feder 92 des Federpaketes 9 gekoppelt, wobei das andere Ende der ersten Feder 9 mit der Verschlusskappe 12 verbunden ist.

Die Gewindestange 11 ist axial fest in Bezug auf das Gehäuse 2 und kann sich zumindest in eine, vorzugsweise in distaler Richtung axialfest am Gehäuseteil 12 (Verschlusskappe) abstützen.

Durch eine Freigabe des Vortriebsglieds 7 wird der ersten Feder 92 erlaubt, dass sie das Vortriebsglied 7 in distale Richtung bewegt. Die erste Feder 92 ist eine spiralförmige Feder, die im Ausgangs- oder Auslieferungszustand des Autoinjektors mit so viel Energie vorgespannt ist, dass sie das in dem Produktbehälter 13 enthaltene Produkt insbesondere vollständig durch Drehen der Gewindestange 11 und mit Verschieben des Vortriebsglieds 7 um einen Ausschütthub H_{A} (nicht gezeigt) aus dem Produktbehälter 13 ausschütten kann. Im Auslieferungszustand der Vorrichtung besteht zwischen dem Kolben 13b und dem distalen Ende des Vortriebsglieds 7 ein Abstand, so dass das Vortriebsglied 7 erst während der Ausführung des Ausschütthubs H_{A} an den Kolben 13b anschlägt und diesen in die Ausschüttrichtung mitnimmt.

In einer vorteilhaften alternativen Ausgestaltung (nicht gezeigt) der Gewindeverbindung zwischen Gewindestange 11 und Vortriebsglied 7 kann insbesondere das Gewinde der Gewindestange 11 eine variable (Gewinde)Steigung aufweisen, wobei in einem ersten Bereich das Gewinde eine grosse Steigung aufweisen kann und in weiteren Bereichen unterschiedlich grosse Steigungen möglich sind. Aus Toleranzgründen kann im Auslieferungszustand des Autoinjektors 0 ein Abstand zwischen Vortriebsglied 7 und Kolben 13b bestehen. Bei der Herstellung wird versucht, den Abstand so klein wie möglich zu halten, damit der Aufprall des Vortriebsgliedes 7 auf den Kolben 13b nicht zu einem Glasbruch führt. Dieser Abstand zwischen Vortriebsglied 7 und Kolben13b wird auch Beschleunigungsweg genannt. Um die Beschleunigung des Vortriebsgliedes 7 im Beschleunigungsweg zu kontrollieren bzw. abzubremsen, und Glasbruchrisiko zu minimieren, wird für den Anfang der Kolbenstangenbewegung ein Gewindeeinlaufweg mit einer grossen Steigung insbesondere auf der Gewindestange 11 und/oder Vortriebsglied 7 gewählt. Bevorzugt ist der Axialabschnitt des Gewindeeinlaufwegs grösser als der Beschleunigungsweg. Des Weiteren können in einer Lagerposition die Axialkräfte, welche insbesondere durch die Gewindeübersetzung aus dem Drehmoment der Feder entstehen durch eine grosse Steigung gering gehalten werden. Das Gewinde bzw. die Gewindesteigung kann über die Länge der Gewindestange 11 und/oder des Vortriebsglieds 7 variieren. Das Gewinde kann ein-oder mehrgängig sein. Vorzugsweise ist das Gewinde zweigängig. Die Steigung kann progressiv oder degressiv sein. Z.B. kann ein weiterer Bereich des Rotationsglieds eine kleinere Steigung aufweisen als der erste Bereich, wobei die grösste Gewindesteigung vorzugsweise nicht selbsthemmend sein kann. Mit solch einem variierenden Gewinde ist es möglich den Abfall des Federkraftmoments zu kompensieren und während der Ausschüttung die Ausschüttkraft in einem konstanten Bereich zu halten. Es ist möglich, dass am Ende der Ausschüttbewegung eine kleine Gewindesteigung gewählt wird und somit die Ausschüttkraft erhöht wird, damit z. B. eine Stopfenreibungskraft welche am Ende der Ausschüttung zunehmen kann, kompensiert wird und eine vollständige Ausschüttung gewährleistet werden kann. Das Rotationsglied und/oder das Vortriebsglied kann mehrere Bereiche mit verschiedenen Gewindesteigungen aufweisen. Z.B. kann das Gewinde eine grosse Gewindesteigung für den Gewindeeinlauf aufweisen und danach einen Bereich mit einer immer kleiner werdenden Gewindesteigung -für eine langsame Ausschüttung- und in einem Bereich mit einer kleinen Gewindesteigung -um ein vollständiges Ausschütten zu gewährleisten-enden. Durch die geeignete Konstruktion der Gewindeverbindung wird also in der Folge die Wahl der Feder 92 im Federpaket 9 vereinfacht, weil der Antriebsstrang Variationen in der Federkraftentfaltung kompensieren kann.

Ferner weist der Autoinjektor 0 ein Halteelement 6 auf, welches in diesem Beispiel zwei Arme 6c aufweist, wobei an jedem Arm 6c ein erstes Eingriffselement 6a und ein zweites Eingriffselement 6b angeordnet ist. Das erste Eingriffselement 6a weist radial zu der Längsachse L hin, wobei das zweite Eingriffselement 6b radial von der Längsachse L weg weist. Das erste Eingriffselement 6a greift in eine Ausnehmung 7a, die von dem Vortriebselement 7 gebildet wird, ein, wodurch eine Bewegung des Vortriebsglieds 7 relativ zu dem Halteelement 6 in die distale Richtung oder in die Ausschüttrichtung verhindert wird. Hierdurch wird die erste Feder 92 in ihrem gespannten Zustand gehalten.

Der Autoinjektor 0 weist ein Schaltmodul 8, 15 auf, welches eine Schalthülse 15 und eine von der Schalthülse 15 umgebene Sperrhülse 8 aufweist, Im Auslieferungszustand der Vorrichtung wird das erste Eingriffselement 6a von dem Innenumfang der Sperrhülse 8, der an dem zweiten Eingriffselement 6b anliegt, in dem Eingriff mit der Ausnehmung 7a gehalten.

Die Schalthülse 15 ist mit dem proximalen Ende 3a der Nadelschutzhülse 3 verbunden oder liegt zumindest an dem proximalen Ende 3a der Nadelschutzhülse 3 an. Eine zweite Feder 10, die vorzugsweise die Schalthülse 15 und die Sperrhülse 8 zumindest teilweise umgibt, stützt sich mit ihrem distalen Ende an der Schalthülse 15 ab. Ein Teil der Schalthülse 15 ist somit zwischen der Nadelschutzhülse 3 und dem distalen Ende der zweiten Feder 10 angeordnet. Die zweite Feder 10 ist eine als Druckfeder wirkende und als Wendelfeder ausgestaltete Feder aus Metall. Die zweite Feder 10 stützt sich mit ihrem proximalen Ende an dem Halteelement 6, insbesondere an einer Abragung 6e, die axial verschiebbar und verdrehfest in das Gehäuse 2 eingreift ab. Die zweite Feder 10 umgibt somit auch den Mechanikhalter 5 zumindest teilweise, vorzugsweise vollständig.

Das Schaltglied 15 weist eine Ausnehmung 15a auf, in welche ein Verriegelungsglied 8a der Sperrhülse 8 eingreift. Das Verriegelungsglied 8a ist sägezahnförmig und ragt radial von der Längsachse L weg. Das Verriegelungsglied 8a ist federnd an einem Arm, welcher von der Sperrhülse 8 gebildet wird, angeordnet. Durch Verschieben der Schalthülse 15 in die proximale Richtung wird die Sperrhülse 8 über den Eingriff des Verriegelungsglieds 8a in die proximale Richtung mitgenommen.

Durch Verschieben der Nadelschutzhülse 3 in die betätigte Position wird die Schalthülse 15 ebenfalls um den Betätigungshub H_{B} (nicht gezeigt) mitgenommen, wodurch die zweite Feder 10 gespannt wird. Wird die Nadelschutzhülse 3 nicht vollständig in die betätigte Position verschoben, kann die zweite Feder 10 die Schalthülse 15 und die Nadelschutzhülse 3 wieder zurück in die Ausgangsposition verschieben, wobei über den Eingriff des Verriegelungsglieds 8a auch die Sperrhülse 8 von der Schalthülse 15 mitgenommen wird.

Zur Verabreichung des Produkts aus dem Produktbehälter 13 wird die Abziehkappe 4 von dem Autoinjektor zusammen mit dem rigid needle shield 14 entfernt. Das distale Ende der Nadelschutzhülse 3 wird an die Einstichstelle eines Patienten angesetzt, wobei das Gehäuse 2 zu der Einstichstelle hin verschoben wird, wodurch sich die Nadelschutzhülse 3 aus ihrer Ausgangsposition um den Betätigungshub H_{B} (nicht gezeigt) in die proximale Richtung relativ zu dem Gehäuse 2 in die betätigte Position bewegt. Hierdurch wird die zweite Feder 10 gespannt, wobei die Schalthülse 15 von der Nadelschutzhülse 3 um den Betätigungshub H_{B} mitgenommen wird. Die Sperrhülse 8 weist eine Ausnehmung bzw. ein distales Ende 8b auf, die durch Verschieben der Sperrhülse 8 um den Betätigungshub H_{B} entlang der Längsachse L auf die Position des zweiten Eingriffselements 6b gebracht wird. Hierdurch wird das erste Eingriffselement 6a aus dem Eingriff mit dem Vortriebsglied 7 mit einer Bewegung quer zu und von der Längsachse L weg bewegt, wobei gleichzeitig das zweite Eingriffselement 6b in den Eingriff mit der Sperrhülse 8, insbesondere deren Ausnehmung 8b bewegt wird. Hierdurch wird das Vortriebsglied 7 für die Bewegung um den Ausschütthub H_{A} (nicht gezeigt) in die Ausschüttrichtung freigegeben.

Da die axialfeste Kopplung zwischen dem Vortriebsglied 7 und dem Halteelement 6 nun aufgehoben ist, kann das Halteelement 6, welches zumindest ein Stück weit relativ zu dem Gehäuse 2 und entlang der Längsachse L bewegbar ist, von der zweiten Feder 10 in die proximale Richtung bewegt werden, wobei das Halteelement 6 über den Eingriff des zweiten Eingriffselements 6b in die Ausnehmung 8b die Sperrhülse 8 um einen Startsignalhub Hs (nicht gezeigt) mitnimmt, wodurch die Sperrhülse 8 an einem Startsignalanschlag, der von dem Mechanikhalter 5 gebildet wird, anschlägt und hierdurch ein akustisches und/oder taktiles Signal ausgibt, welches dem Anwender der Vorrichtung signalisiert, dass die Produktausschüttung gestartet wurde.

Da das zweite Eingriffsglied 6b immer noch in der Ausnehmung 8b der Sperrhülse 8 ansteht wird hierdurch das Haltelement 6 daran gehindert, sich weiter in die proximale Richtung relativ zu dem Gehäuse 2 oder der Sperrhülse 8 zu bewegen. Das zweite Eingriffsglied 6b wird von dem Außenumfang des Vortriebsglieds 7 in den Eingriff mit der Ausnehmung 8b gehalten, wenn das Vortriebsglied 7 um seinen Ausschütthubs H_{A} bewegt wird.

Am Ende des Ausschütthubs H_{A} gibt das Vortriebsglied 7 das erste Eingriffsglied 6a für eine Bewegung insbesondere zur Längsachse L hin frei, wodurch das zweite Eingriffsglied 6b aus dem Eingriff mit der Ausnehmung 8b der Sperrhülse 8 bewegt wird, so dass die zweite Feder 10 das Halteelement 6 entgegen die Ausschüttrichtung, d. h. in die proximale Richtung beschleunigt, so dass beim Auftreffen des Halteelements 6 auf dem Endsignalanschlag 5e ein akustisches und/oder taktiles Signal erzeugt wird.

Durch Abnehmen des Autoinjektors von der Injektionsstelle kann die zweite Feder 10 die Schalthülse 15 und die Nadelschutzhülse 3 aus der betätigten Position in die Nadelschutzposition um den Nadelschutzhub H_{N} bewegen. Ein Verriegelungsglied 8a an der Sperrhülse 8, welches in Eingriff mit der Schalthülse 15 gelangt, verhindert sodann ein erneutes Zurückschieben der Nadelschutzhülse 3.

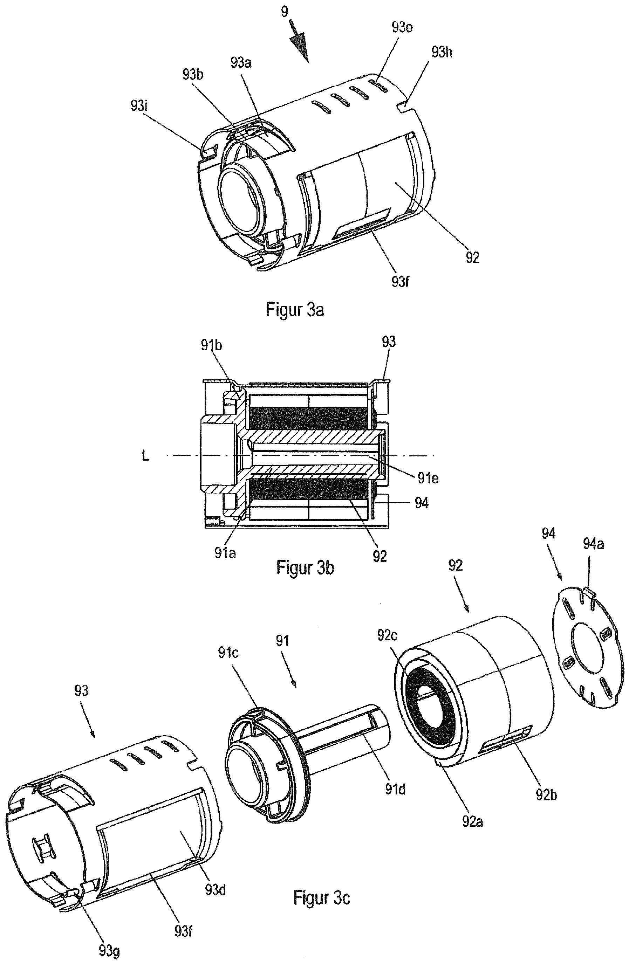

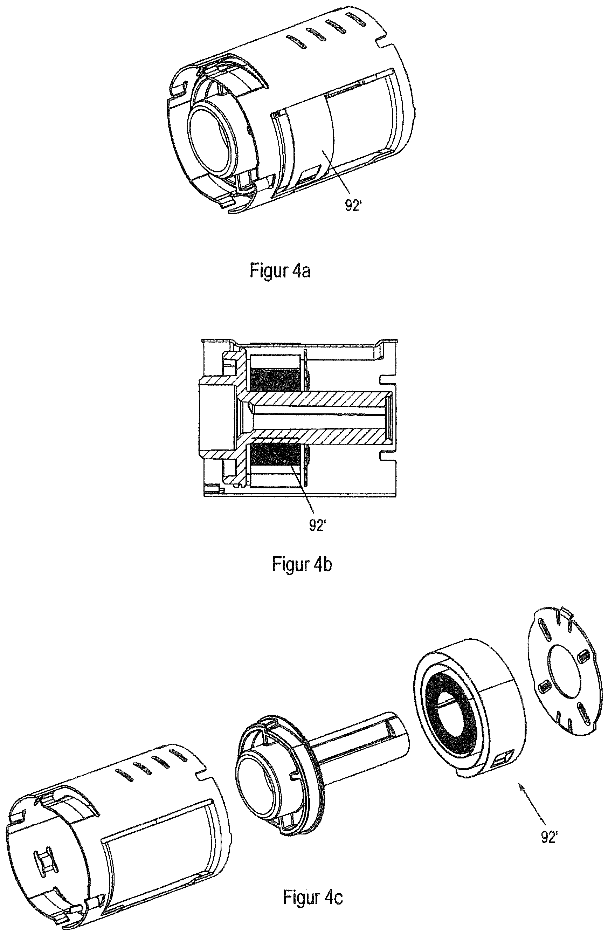

Die Figuren 3a-3c zeigen eine erste Ausführungsform eines erfindungsgemässen Federpaketes, wie es zum Beispiel in Autoinjektor 0 oder auch in den unten beschriebenen Autopen 100 eingebaut werden könnte. Die Figuren 4a-4c zeigen eine Ausführungsform mit einer alternativen Spiralfeder.

Figur 3a zeigt eine perspektivische Ansicht des Federpakets 9, Figur 3b einen Längsschnitt durch das Federpaket 9 und Figur 3c zeigt eine explodierte Darstellung des Federpaketes 9. Das Federpaket 9 besteht grundsätzlich aus vier Einzelteilen, dem Federschaft 91 mit Schaft 91a und Flansch 91b, der Spiralfeder 92, der Federhülse 93 und dem Federhülsendeckel 94. Das innere Ende der Spiralfeder 92c ist mittels Lasche in der Federverankerung 91d befestigt, dabei kann die Feder 92 in axialer Richtung L beweglich gelagert sein. Das äussere Ende der Feder 92a ist mittels Lasche 92b an der Haltevorrichtung 93d der Federhülse 93 befestigt. Auch hier kann eine Bewegung in axiale Richtung L zwischen Federhülse 93 und Feder 92 möglich sein. Der Flansch 91b weist einen oder mehrere radiale Anschläge 91c auf, in welche der Sperrschnapper 93a mittels Zahn 93g eingreifen kann. Die Sperrrichtung ist Anschlag 91c/Zahn 93g ist dabei so gewählt, dass eine Entspannung der Feder 92 verhindert werden kann. Der Federschaft 91 weist eine Bohrung oder Öffnung 91e in axialer Richtung L auf. In der Öffnung kann das proximale Ende der Gewindestange 11 drehfest gelagert werden. Während der der Flansch 91b in distaler Richtung das Federpaket abschliesst, so dient der Federhülsendeckel 94 dem Abschluss in proximale Richtung, wobei der Federhülsendeckel 94 mittels der in radialer Richtung abragenden Fixierelemenente 94a in den Öffnungen 93e der Federhülse befestigt werden kann. Das Federpaket aus den Figuren 4a-4c unterscheidet sich vom Federpaket aus den Figuren 3a-3c hinsichtlich der Feder 92'. Das Band der Spiralfeder 92' ist schmaler als dasjenige der Spiralfeder 92. Der Vergleich zwischen den beiden Varianten zeigt einen der Vorteile des erfindungsgemässen Aufbaus der Federpaketes 9. Ohne dass sich konstruktive Änderungen an Federschaft, Federhülse oder Federhülsendeckel ergeben, lassen sich Federn mit unterschiedlicher Grösse einsetzen. In der Folge muss das Verabreichungsgerät, zum Beispiel der Autoinjektor 0 oder der Autopen 100, konstruktiv nicht angepasst werden. So kann bei gleichbleibender Konstruktion des Verabreichungsgerätes eine Anpassung der Kraft, welche beim Verabreichen eines bestimmten Medikaments wirken soll, stattfinden, indem lediglich eine spezifisch angepasste Feder eingesetzt wird, wobei alternativ auch mehrere Federn eingesetzt werden, welche die spezifische Anpassung ergeben. Die an verschiedenen Positionen abgebrachten Öffnungen 93e auf der Federhülse 93 erlauben es, dass der Federhülsendeckel 94 angepasst an die Bandbreite der Feder 92 in der Federhülse 93 befestigt werden kann, wobei beim alternativen Einsatz von mehreren Fedem auch mehrere Deckel eingesetzt werden können.

Die Federhülse 93 weist in ihrem distalen Bereich den Sperrschnapper 93a sowie den Steuerarm 93b auf. Beide sind Teile desselben flexiblen Armes, so dass eine Bewegung des freien Endes des Steuerarmes 93b in radiale Richtung eine Bewegung des freien Endes des Sperrschnappers 93a, insbesondere des Zahnes 93g, in dieselbe Richtung zur Folge hat.

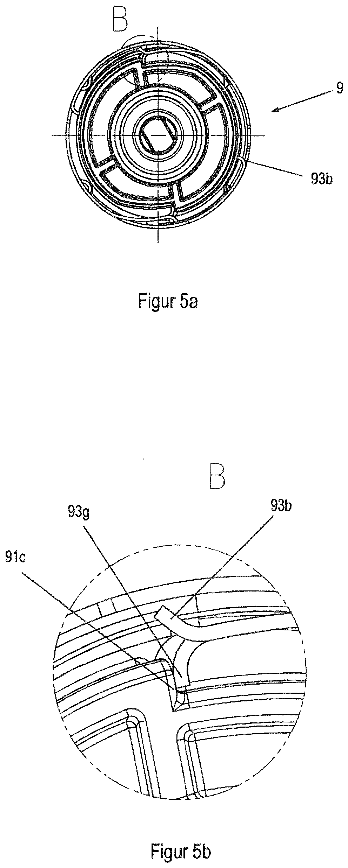

Die Spiralfeder 92 kann durch relative Rotation der Federhülse 93 und dem Federschaft 91 gespannt werden. Im vorliegenden Beispiel (Figur 3) und von proximal her gesehen, wird die Federhülse 93 im Gegenuhrzeigersinn relativ zum Federschaft 91 rotiert, um die Feder 92 in Wickelrichtung zu spannen, Ist die Feder 92 gespannt, so kann die Entspannung der Feder 92 über den Eingriff von Sperrschnapper 93a in den radialen Anschlag 91c verhindert werden. In einer bevorzugten Variante ist die Federhülse aus einem metallischen Blech geformt, bei welchem der Sperrschnapper 93a über plastische Deformation mit dem radialen Anschlag in Eingriff gebracht werden kann. Somit kann im Federpaket potentielle Energie gespeichert werden und das Paket kann bei gespannter Feder auch als Schüttgut transportiert oder aufbewahrt werden. Die Figuren 5a und 5b zeigen eine Ansicht des Federpaketes 9 aus distaler Richtung, wobei Figur 5b ein vergrösserter Ausschnitt aus Figur 5a ist. Vor allem in Figur 5b ist der Eingriff zwischen Zahn 93g und radialem Anschlag 91c sehr gut ersichtlich.

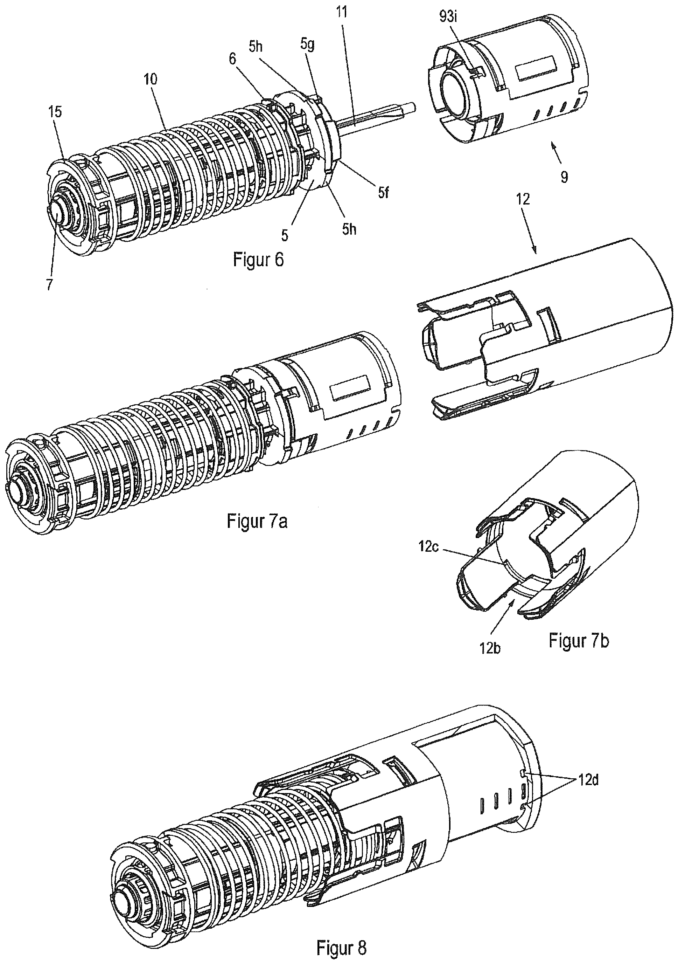

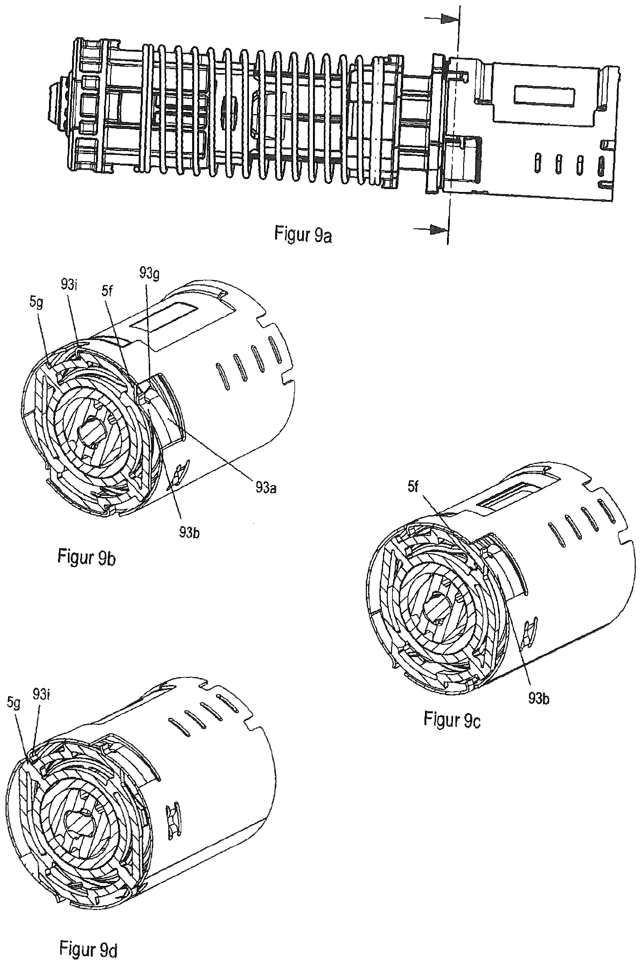

Die Figuren 6 bis 8 zeigen schrittweise den Einbau des Federpaketes 9 in den Autoinjektor 0. Ein einem ersten Schritt wird das Federpaket mit der vormontierten Antriebseinheit (linke Seite Figur 6) des Autonjektors 0 zusammengebracht, dabei ist wichtig zu erwähnen, dass insbesondere das Vortriebsglied 7 bereits verdrehgesichert im Mechanikhalter 5 gelagert ist und auch das Halteelement 6 so montiert ist, dass das Vortriebsglied 7 nicht in distale Richtung bewegt werden kann. Dabei wird das Federpaket 9 auf das proximale Ender der Gewindestange 11 gesteckt. Der Querschnitt des proximalen Endes der Gewindestange 11 ist dabei nicht kreisförmig und die Öffnung 91e des Federschaftes 91 ist komplementär dazu ausgebildet, so dass beide Teile zu einander verdrehgesichert werden. Beim Aufstecken muss dabei auf die Orientierung des Mechanikhalters 5 geachtet werden, da das Freigabeelement 5f rotativ vor dem Steuerarm 93b zu liegen kommen soll, siehe dazu auch Figur 9b, welche die Situation bei aufgestecktem Federpaket 9 angeschnitten darstellt. In Figur 7a ist das Federpaket vollständig mit der vormontierten Antriebseinheit zusammengebracht.

Nun wird die Verschlusskappe 12 über das Federpaket 9 hinweg mit der vormontierten Antriebseinheit verbunden. Kurz zusammengefasst passiert Folgendes: der Mechanikhalter 5 und die Verschlusskappe 12 werden über einen Bajonettverschluss fest miteinander verbunden. Weiter wird die Federhülse 93 verdrehfest mit der Verschlusskappe 12 verbunden, es wird der Sperreingriff zwischen Sperrschnapper 93a und radialem Anschlag 91c gelöst und in einem letzten Schritt wird eine Rotation der Federhülse 93 in Richtung einer Entspannung der Feder 92 durch Eingriff des Block 93i in den radialen Anschlag 5g des Mechanikhalters 5 unterbunden.

Für den Bajonettverschluss zwischen Mechanikhalter 5 und Verschlusskappe 12 sind auf dem Mechanikhalter 5 Bajonettnocken 5h angeordnet (im vorliegenden Fall sind es vier, wobei grundsätzlich mindestens eine gebraucht wird). Auf der Innenfläche der Verschlusskappe 12 sind entsprechend komplementär Bajonettnuten 12b/c angeordnet, wobei die Nuten zumindest aus einem axial verlaufenden Anteil 12b sowie einem entlang des Umfangs verlaufenden Anteil 12c bestehen. Die Verschlusskappe 12 wird nun so auf die vormontierte Antriebseinheit geschoben, dass die Bajonettnocken 5h in die Nuten 12b eingeschoben werden. Dabei wird die Verschlusskappe soweit auf die Antriebseinheit aufgeschoben, dass die Verdrehsicherungen 93h der Federhülse in Eingriff mit Verdrehsicherungen 12d der Verschlusskappe 12 gelangen, womit Verschlusskappe 12 und Federhülse 93 verdrehsicher verbunden sind. Nun wird die Verschlusskappe 12 relativ zum Mechanikhalter 5 verdreht, so dass sich die Bajonettnocken 5h nun in den Nuten 12c weiterbewegen. Aufgrund der Verdrehsicherung zwischen Verschlusskappe 12 und Federhülse 93 wird auch die Federhülse 93 relativ zum Mechanikhalter 5 verdreht. Das führt dazu, dass die Freigabeelemente 5f die zugeordneten Steuerarme 93b radial anheben und dadurch auch die zugehörige Verbindung zwischen Sperrschnapper 93a und radialem Anschlag 91c gelöst wird. In vorteilhafter Konsequenz unterstützt eine allfällige Vorspannung der Spiralfeder 92 die Verschlussbewegung des Bajonettverschlusses bis der Block 93i in Anschlag mit dem radialen Anschlag 5g des Mechanikhalters gelangt, womit die Verschlussbewegung abgeschlossen ist. In diesem Zustand wird das Drehmoment der Spiralfeder 92 auf der Aussenseite über den radialen Anschlag 5g des Mechanikhalters 5 gehalten. Auf der Innenseite wird das Drehmoment über den Federschaft 91 auf die Gewindestange 11 übertragen. Die Gewindestange 11 ist ihrerseits - wie beschrieben - über eine Gewindeverbindung mit dem Vortriebsglied 7 verbunden, welches seinerseits verdrehgesichert mit dem Mechanikhalter 5 verbunden, so dass das Drehmoment in eine Kraft übersetzt wird, welche axial in distale Richtung auf das Vortriebsglied wirkt. Wie in Figur 2b gezeigt, wird das Vortriebsglied 7 durch den Eingriff des Eingriffelementes 6a in die Ausnehmung 7a vor Auslösung des Autoinjektor 0 an einer Bewegung in distale Richtung gehindert. Die Spiralfeder 92 ist also auch in dieser Phase des Zusammenbaus des Autoinjektors 0 stabil gelagert, ohne dass eine allfällig vorhandene Vorspannung verloren ginge. Die Endmontange des Autoinjektors 0 kann so weiter fortgesetzt werden, wie es dem Fachmann geläufig ist.

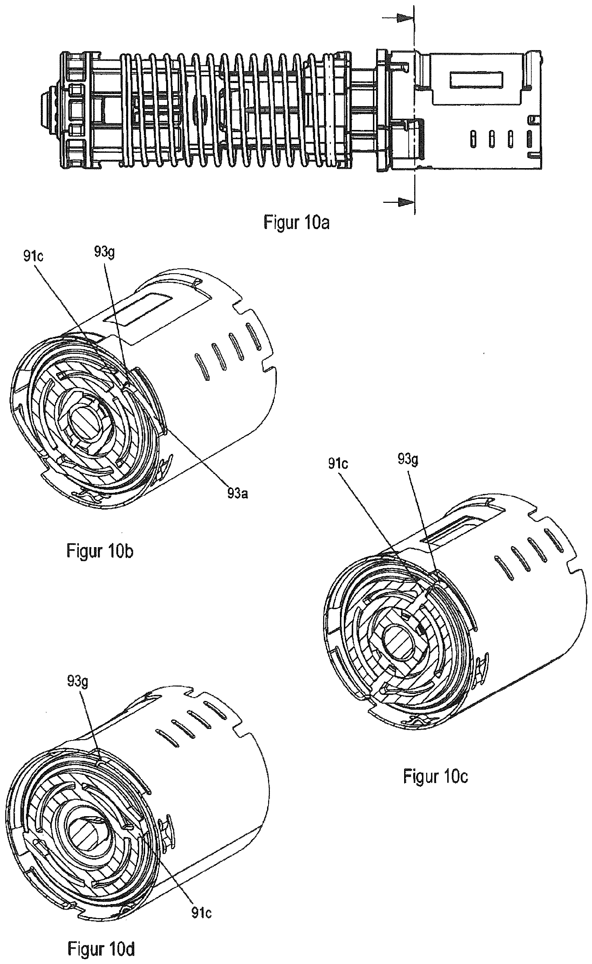

Die obig beschriebenen Phasen des Zusammenbaus von vormontierter Antriebseinheit, Federpaket und Verschlusskappe ist ergänzend auch den Figuren 9a-9d sowie 10a-10d dokumentiert. In den 9b-9d wird gezeigt wie das Freigabeelement 5f dem Steueram 93b anhebt, wodurch die Verbindung zwischen Sperrschnapper 93a und radialem Anschlag 91c gelöst wird, und anschliessend der radiale Block 93i der Federhülse 93 in Eingriff mit dem radialen Anschlag 5g des Mechanikhalters 5 kommt. Die Figuren 10b-10d zeigt dieselben Phasen wie Figuren 9b-9d, wobei der Querschnitt auf Höhe des Sperrschnappers 93a erfolgt.

Bezugnehmend auf die Figuren 11 bis 12b wird im Folgenden eine zweite mögliche erfindungsgemässe Verabreichungsvorrichtung mit erfindungsgemässem Federpaket beschrieben, basierend auf einem automatischen Injektionspen 100, wie er in Figur 11 dargestellt ist, die Verabreichungsvorrichtung 100 wird folgend vereinfacht als Autopen bezeichnet. Es wird an dieser Stelle die WO2015/135083 referenziert und vollständig in die vorliegende Anmeldung integriert, da sie den Autopen 100 abgesehen vom Federpaket ebenfalls konzeptionell beschreibt.

Das Federpaket 109 ist analog zu Federpaket 9 auf gebaut, entsprechend werden korrespondierende Teile und Merkmale mit einem Bezeichner plus 100 markiert. Als Beispiel sei die Spiralfeder 192 des Autopens 100 erwähnt, welche bei Autoinjektor 0 mit der Spiralfeder 92 korrespondiert. Gleiches gilt für analoge Teile in der Verabreichungsvorrichtung.

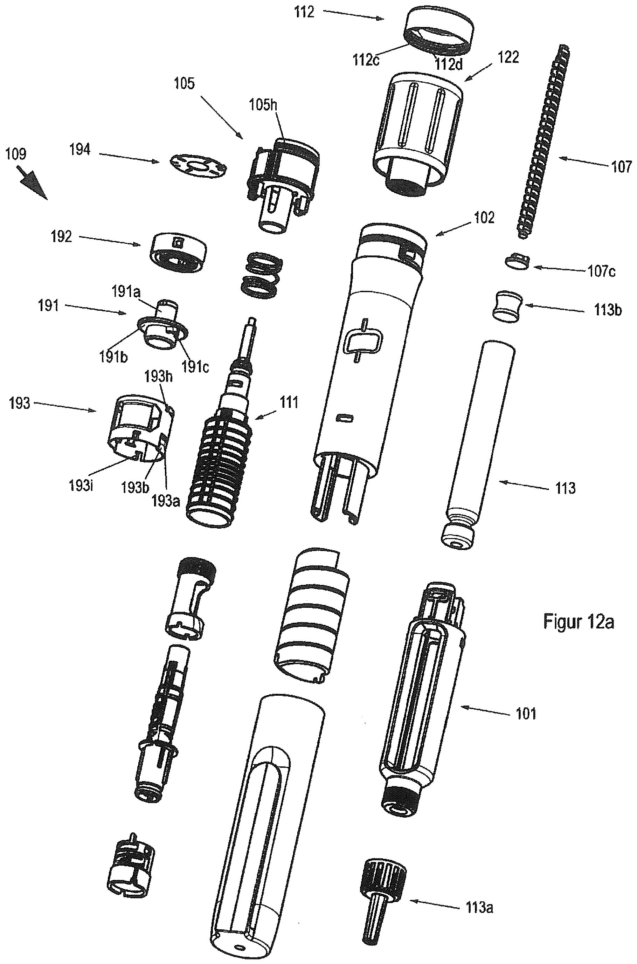

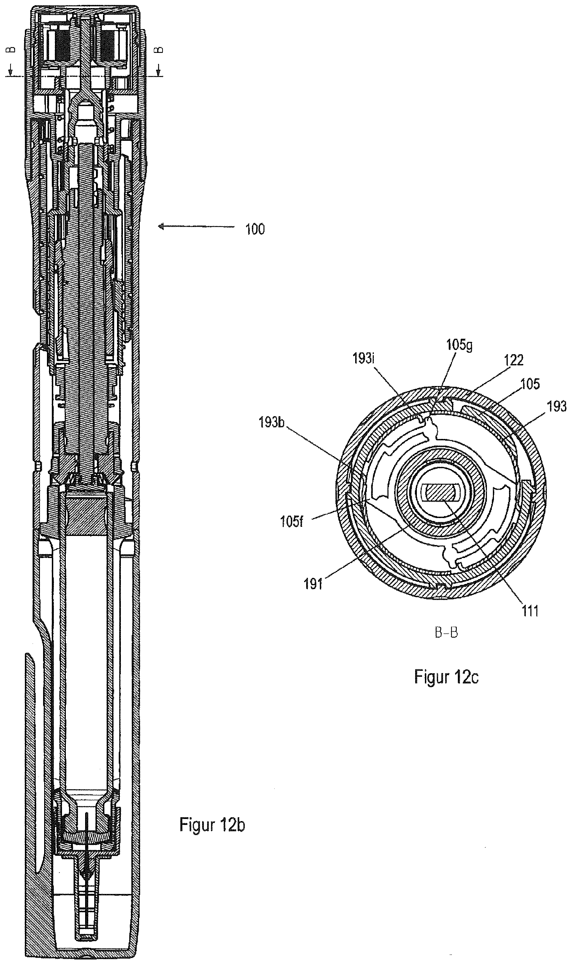

Der Autopen 100 is so ausgelegt, dass von der benutzenden Person mehrere Injektionen mit variabler Dosis verabreicht werden können. Wenn der Medikamentenbehälter 113 leer wird der Autopen 100 als Ganzes entsorgt. Die Spiralfeder 192 des Federpaketes 109 ist dabei so ausgelegt, dass sie vorgespannt ist und der ganze Medikamentenbehälter 113 mit der gespeicherten Energie geleert werden kann. Das Federpaket 109 ist bei Autopen 100 im Dosiseinstellglied 122 lokalisiert. Dabei ist der Federschaft 191 mit dem Antriebsglied 111 drehfest verbunden. Im montierten Zustand, wie in Figur 12b gezeigt, verhindert der Eingriff des radialen Blocks 193i der Federhülse 193 in den radialen Anschlag 105g der Federaufnahme 105 ein Entspannen der Spiralfeder.

Die Montage des Federpaketes 109 und die Freigabe des Drehmoments aus der Spiralfeder 192 erfolgt sinngemäss fast gleich wie beim Autoinjektor 0. Während der Montage übernimmt die Federaufnahme 105 die Aufgaben des Mechanikhalters 5, das Antriebsglied 111 entspricht der Gewindestange 11 und Deckel 112 entspricht der Verschlusskappe (oder Gehäuseteil) 12. Zumindest Dosierelement 122, Federaufnahme 105 und Antriebsglied 111 sind so vormontiert, dass alle drei Teile zueinander verdrehgesichert sind.

Die Geometrie des Deckels 112 hat einen Unterschied bei der Montage zur Folge. Statt über einen Bajonettverschluss wird der Deckel 112 über eine Schnappverbindung mit der Federaufnahme 105 verbunden. Dazu ist auf dem Umfang der Federaufnahme 105 eine Nut 105h angeordnet und auf der Innenfläche des Deckels 112 komplementär dazu eine Rippe 112c. Funktionell entspricht die Rippe 112c der Bajonettnut 12c des Autoinjektors 0. Bei der Montage wird zuerst das Federpaket in die Federaufnahme 105 geschoben und der Federschaft 191 verdrehgesichert mit dem Antriebsglied 111 verbunden. Danach wird der Deckel 112 auf die Federaufnahme 105 aufgeschnappt (was funktionell dem ersten Teil des Bajonettverschlussvorganges beim Autoinjektor 0 entspricht), wobei die Verdrehsicherungselemente 112d des Deckels 112 so ausgerichtet werden müssen, dass sie in die Verdrehsicherungselemente 193h der Federhülse 193 eingreifen können. Nach dem der Deckel 112 sauber aufgeschnappt hat, kann der Deckel 112 relativ zur Federaufnahme 105 verdreht werden, wobei die Freigabeelemente 105f den zugehörigen Steuerarm 193b radial nach aussen bewegen und damit den Eingriff von Sperrschnapper 193a und radialem Anschlag 191c am Federschaft 191 lösen. Das aus der Spiralfeder 192 wirkende Drehmoment bewirkt nun, dass der Deckel 112 zusammen mit der Federhülse 193 relativ zur Federaufnahme 105 gedreht werden bis der Block 1931 der Federhülse 193 in Eingriff mit dem radialen Anschlag 105 der Federaufnahme gelangt

Für den Autopen 100 sind auch einfachere Varianten der Montage vorteilhaft auszugestalten. So kann zum Beispiel der Eingriff zwischen Sperrschnapper 193a und dem radialen Anschlag 191c in einer Variante auch ohne den Deckel 112 gelöst werden. Dies kann so geschehen, dass, nachdem das Federpaket 109 in die Federaufnahme 105 eingeschoben worden ist, direkt die Federhülse 193 relativ zur Federaufnahme verdreht wird und die Freigabeelemente 105f, wie oben beschrieben, so dann den Steuerarm betätigen. Der Deckel 112 könnte sodann im Anschluss auf die Federaufnahme 105 aufgeschnappt werden. Eine Verdrehsicherung 112d/193h wäre in der Folge dann obsolet.

Weiter wäre in einer weiteren vorteilhaften Vereinfachung auch denkbar, dass der Steuerarm schon beim Einschieben des Federpakets 109 in die Federaufnahme 105 betätigt würde und so keine relative Rotation zwischen Federhülse 193 und Federaufnahme 105 zur Freigabe des Drehmoment nötig wäre.

**Bezugszeichenliste**

| | | | |
|---|---|---|---|
| 0 | Autoinjektor, Injektionsgerät | 8 | Sperrhülse |
| | | 8a | Verriegelungsglied |
| 1 | Spritzenhalter | 8b | erste Ausnehmung /distales Ende |
| 1a | Abragung | 8c | Sperrarm |
| 1b | Schulter | | |
| 1c | Verrastung | 9 | Federpaket |
| | | 91 | Federschaft |
| 2 | Gehäuse | 91a | Schaft |
| 2a | Ausnehmung | 91b | Flansch |
| 2b | Verrastöffnung | 91c | radialer Anschlag |
| | | 91d | Federverankerung |
| 3 | Nadelschutzhülse | 91e | Öffnung |
| 3a | proximales Ende | 92 | Spiralfeder |
| | | 92' | alternative Spiralfeder |
| 4 | Abziehkappe | 92a | äusseres Ende |
| 4a | Schnapphaken | 92b | Haltelasche |
| 4f | Nadelschutzkappenentfemer | 92c | inneres Ende mit Haltelasche |
| | | 93 | Federhülse |
| 5 | Mechanikhalter | 93a | Sperrschnapper |
| 5c | Haltefederabschnitt | 93b | Steuerarm |
| 5e | Endsignalanschlag | 93c | Sperrelement |
| 5f | Freigabeelement | 93d | Haltevorrichturig |
| 5g | radialer Anschlag | 93e | Öffnung |
| 5h | Bajonettnocke | 93f | Haltekante |
| | | 93g | Zahn |
| 6 | Halteelement | 93h | Verdrehsicherung |
| 6a | erstes Eingriffselement/-glied | 93i | radialer Block |
| 6b | zweites Eingriffselement/-glied | 94 | Federhülsendeckel |
| 6c | Arm | 94a | Fixierelement |
| 6e | Abragung | | |
| | | 10 | Nadelschutzfeder |
| 7 | Vortriebsglied/Kolbenstange | | |
| 7a | Ausnehmung | 11 | Gewindestange |
| 7b | Gewindesegment | | |
| | | 12 | Verschlusskappe |
| 12a | Rastglied | 113b | Karpulenstopfen |
| 12b | Bajonettnut, axial verlaufender Teil | | |
| 12c | Bajonettnut, umfänglich verlaufender Teil | 109 | Federpaket |
| 12d | Verdrehsicherung | 191 | Federschaft |
| | | 191a | Schaft |
| 13 | Produktbehälter / Spritze | 191b | Flansch |
| 13a | Kanüle / Nadel | 192 | Spiralfeder |
| 13b | Kolben / Stopfen | 193 | Federhülse |
| 13c | Fingerflansch | 193a | Sperrschnapper |
| | | 193b | Steuerarm |
| 14 | Nadelschutzkappe | 193h | Verdrehsicherung |
| | | 193i | radialer Block |
| 15 | Schalthülse | 194 | Federhülsendeckel |
| 15a | Ausnehmung | | |
| | | 122 | Dosiseinstellelement |
| 100 | Autopen, Injektionsgerät | | |
| 101 | Karpulenhalter | | |
| 102 | Gehäuse | L | Längsachse |
| 105 | Federaufnahme | | |
| 105f | Freigabeelement | | |
| 105g | radialer Anschlag | | |
| 105h | Nut | | |
| 107 | Kolbenstange | | |
| 107c | Kolbenstangenflansch | | |
| 111 | Antriebsglied | | |
| 112 | Deckel | | |
| 112c | Rippe | | |
| 112d | Verdrehsicherung | | |
| 113 | Karpule | | |
| 113a | Injektionsnadel | | |

## Patentansprüche

1. Federpaket (9) für eine Verabreichungsvorrichtung, umfassend
eine Feder (92), welche als Trieb-oder Spiralfeder ausgebildet ist,
einen Federschaft (91) zur Montage auf einer vormontierten Antriebseinheit der Verabreichungsvorrichtung,
wobei die Antriebseinheit eine Federaufnahme und ein beweglich in der Federaufnahme angeordnetes Antriebsglied umfasst, wobei der Federschaft (91) einen fest angebrachten Flansch (91b) umfasst, wobei ein inneres Ende der Feder (92) zumindest drehfest am Federschaft (91) angebracht ist,
eine Federhülse (93), welche den Mantelbereich der Feder (92) zumindest teilweise umgibt, wobei das äussere Ende (92a) der Feder (92) drehfest mit der Federhülse (93) verbunden ist,
einen scheibenartigen Federhülsendeckel (94), welcher fest an der Federhülse (93) oder dem Federschaft (91) angebracht werden kann, wobei der Tellerdurchmesser des Federhülsendeckels (94) gleich oder kleiner ist als derjenige der Federhülse (93),
wobei der Federschaft (91) eine Achse definiert, ein proximales und ein distales Ende aufweist und sich Flansch (91b) und Federhülsendeckel (94) radial von der Achse weg erstrecken,
wobei der Flansch (91b) in der Nähe des distalen Endes des Federschaftes (91) angeordnet ist
***dadurch gekennzeichnet, dass***
an der Peripherie des Flansches (91b) mindestens ein radialer Anschlag (91c) angeordnet ist, und
an der Federhülse (93) komplementär zum mindestens einen radialen Anschlag (91c) mindestens ein als Sperrschnapper (93a) ausgebildetes Blockiermittel angeordnet ist, so dass bei einem Eingriff von Sperrschnapper (93a) und radialem Anschlag (91c) die Federhülse (93) und der Federschaft (91) relativ zueinander und lösbar verdrehgesichert werden,
wobei die Feder (92) im Federpaket (9) vorgespannt ist, so dass ein Drehmoment auf den Federschaft (91) oder die Federhülse (93) ausgeübt wird,
wobei durch die Verdrehsicherung eine Federspannung der vorgespannten Feder (92) gehalten werden kann,
wobei durch eine Bewegung des Sperrschnappers (93a) die Verdrehsicherung gelöst werden kann, so dass in einer ausgelösten Position eine relative Drehung zwischen Federschaft (91) und Federhülse (93) möglich ist, so dass das Drehmoment von der Feder (92) nach ausserhalb des Federpakets (9) geleitet werden kann.

2. Federpaket (9) nach Anspruch 1, wobei die axiale Positionierung der Feder (92) durch die feste Position des Flansches (91b), sowie eine variable axiale Fixierung des Federhülsendeckels (94) definiert wird und dadurch Federn mit unterschiedlicher axialer Ausdehnung im Federpaket (9) eingesetzt werden können.

3. Federpaket (9) nach Anspruch 2, wobei der Federhülsendeckel (94) in die Federhülse (93) hineinverschoben werden kann, wobei der Federhülsendeckel (94) an seinem Umfang mindestens ein Schnappelement aufweist, und die Federhülse (93) dazu komplementär mindestens eine Aufnahme für das mindestens eine Schnappelement aufweist, um den Federhülsendeckel (94) an der Federhülse (93) zu befestigen.

4. Federpaket (9) nach Anspruch 3, wobei die Federhülse (93) an mehreren axialen Positionen mindestens eine Aufnahme für das mindestens eine Schnappelement des Federhülsendeckels (94) umfasst, so dass der Federhülsendeckel (94) in verschiedenen axialen Positionen an der Federhülse (93) befestigt werden kann.

5. Federpaket (9) nach einem der Ansprüche 1 bis 4, **dadurch gekennzeichnet, dass** die Feder (92) aus spiralig aufgerolltem Bandmaterial besteht, bevorzugt aus metallischem Bandmaterial, besonders bevorzugt aus bandförmigem Stahl.

6. Federpaket (9) nach einem der vorhergehenden Ansprüche, wobei die Federhülse (93) koaxial zum Federschaft (91) angeordnet ist, **dadurch gekennzeichnet, dass** an der Federhülse (93) zumindest ein flexibler Arm angeordnet ist, welcher sich in Umfangsrichtung der Federhülse (93) erstreckt, welcher mit einem Ende fest an der Federhülse (93) angebracht ist und am anderen Ende in radiale Richtung ausgelenkt werden kann und wobei am freien Ende das Blockiermittel, insbesondere in der Form eines Zahnes (93g) angeordnet ist, welcher durch Auslenkung des zumindest einen flexiblen Armes in Eingriff mit dem radialen Anschlag (91c) gebracht werden kann oder aus dem Eingriff gelöst werden kann, so dass der flexible Arm zusammen mit dem Blockiermittel einen Sperrschnapper (93a) bilden kann.

7. Federpaket (9) nach einem der vorhergehenden Ansprüche, wobei der Federschaft (91) eine axial ausgebildete Halterippe aufweist, in welcher das als Haltelasche ausgebildete innere Ende (92c) der Feder (92) drehfest verankert werden kann, und wobei die Federhülse (93) eine axial orientierte Haltestruktur aufweist, an welcher das als Haltelasche ausgebildete äussere Ende (92c) der Feder (92) drehfest verankert werden kann.

8. Federpaket (9) nach Anspruch 6 und 7, wobei die Feder (92) durch relative Drehung der Federhülse (93) zum Federschaft (91) vorgespannt werden kann, und wobei diese Vorspannung durch einen Eingriff von radialen Anschlag (91c) und Blockiermittel gehalten werden kann, und wobei die Vorspannung einem Drehmoment von 1 bis 100 Nmm entsprechen kann, bevorzugt einem Drehmoment von 30 bis 80 Nmm entsprechen kann und besonders bevorzugt einem Drehmoment von 60 bis 70 Nmm entsprechen kann.

9. Federpaket (9) nach den Ansprüche 6 oder 8, wobei am freien Ende des Armes axial in distale Richtung zum Blockiermittel versetzt ein Steuerarm (93b) angeordnet ist, und wobei bei radialer Auslenkung des Steuerarmes (93b) auch das Blockiermittel entsprechend radial bewegt wird und umgekehrt.

10. Verabreichungsvorrichtung zur Verabreichung eines fluiden Produktes, die Verabreichungsvorrichtung umfassend
eine Längsachse,
ein Gehäuse (2) mit einem Mechanikhalter (5) welcher fest mit dem Gehäuse (2) verbunden ist,
eine Auslösevorrichtung,
einen Produktbehälter (13), insbesondere in der Form einer vorbefüllten Spritze oder Karpule, welcher zumindest axial fest in einem Teil des Gehäuses (2) angeordnet ist, wobei im Produktbehälter (13) ein axial verschiebbarer Stopfen angeordnet ist, durch dessen Verschiebung in distale Richtung Produkt aus dem Produktbehälter (13) ausgeschüttet werden kann,
ein Federpaket (9) nach Anspruch 9, in welchem Energie für das automatische Ausschütten von Produkt gespeichert ist, wobei das Federpaket (9) operativ mit der Auslösevorrichtung verbunden ist, und wobei die Federhülse (93) drehfest mit dem Gehäuse (2) verbunden ist,
eine koaxial zur Längsachse angeordnete Gewindestange (11), welche drehfest mit dem Federschaft (91) verbunden ist,
eine koaxial zur Längsachse angeordnete Kolbenstange (7), welche axial verschiebbar und drehfest im Mechanikhalter (5) geführt wird, wobei die Kolbenstange (7) mit dem Stopfen des Produktbehälters (13) so wechselwirken kann, dass bei axialen Bewegung der Kolbenstange (7) in distale Richtung auch der Stopfen in distale Richtung verschoben werden kann,
wobei die Kolbenstange (7) hülsenartig aufgebaut ist und über Gewindeelemente auf einer inneren Oberfläche der Kolbenstange (7) in Gewindeeingriffe mit der Gewindestange (11) ist, so dass eine Rotation der Gewindestange (11), eine axiale Verschiebung der Kolbenstange (7) zur Folge hat.

11. Verabreichungsvorrichtung, nach Anspruch 10, wobei der Mechanikhalter (5) mit einem Gehäuseteil (12) durch einen Bajonettverschluss verbunden wird, indem Führungselemente des Mechanikhalters (5) als Bajonettnocken (5h) ausgebildet sind und eine Führung entlang des Umfangs des Gehäuseteils (12) als Bajonettnuten (12c) ausgeführt ist, so dass die Bajonettnocken (5h) in die Bajonettnuten (12c) eingreifen können.

12. Verabreichungsvorrichtung nach Anspruch 10 oder 11, wobei es sich bei der Verabreichungsvorrichtung um einen Autoinjektor (0) oder einen Injektionspen mit automatischer Verabreichung handelt.

13. Verabreichungsvorrichtung nach Anspruch 10 oder 11, wobei es sich bei der Verabreichungsvorrichtung um ein ,patch'-Gerät handelt.

14. Verfahren zur Montage eines Federpaketes (9) mit einer vorgespannten Trieb-oder Spiralfeder (92) in eine Verabreichungsvorrichtung zur Verabreichung eines fluiden Produktes, insbesondere eine längliche Injektionsvorrichtung, mit einem Gehäuse (2) und einem gehäusefesten Mechanikhalter (5) umfassend zumindest folgende Schritte:
a) das Federpaket (9) wird axial auf eine vormontierte Antriebseinheit der Verabreichungsvorrichtung, welche den Mechanikhalter (5) umfasst, geschoben,
wodurch ein drehbares in der Antriebseinheit vorhandenes Antriebsglied mit einem Federschaft (91) des Federpaketes (9) verdrehsicher verbunden wird, und
wodurch sich ein fest am Mechanikhalter (5) angeordnetes Freigabeelement vor einen Steuerarm (93b) des Federpaketes (9) schiebt,
b) ein Gehäuseteil (12) oder Abschlussteil der Verabreichungsvorrichtung wird in distale Richtung axial über das Federpaket (9) geschoben,
wodurch die äussere Mantelfläche des Federpaketes (9) verdrehgesichert mit dem Gehäuseteil (12) oder Abschlussteil verbunden wird,
c) das Gehäuseteil (12) oder Abschlussteil wird relativ zu Federpaket (9) und vormontierter Antriebseinheit verdreht,
wodurch die Führungselemente des Mechanikhalters (5) in Führungen des Gehäuseteils (12) oder Abschlussteil geführt werden, wobei die Führung entlang des Umfangs des Gehäuseteils (12) oder Abschlussteils verläuft,
wobei durch die Verdrehung das Freigabeelement den Steuerarm (93b) in radiale Richtung nach aussen bewegt und somit ein im Federpaket (9) vorhandenes Drehmoment freigibt, wodurch eine weitere relative Rotation zwischen Gehäuse- oder Abschlussteil und dem Mechanikhalter (5) evoziert bis ein radialer Block des Federpaketes (9) in Eingriff mit einem radialen Anschlag der Mechanikhalter (5) gerät und so eine kraftschlüssige Verbindung zwischen Federpaket (9) und Mechanikhalter (5) erzeugt wird.

## Claims

1. A spring assembly (9) for an administration device, comprising
a spring (92) in the form of a drive or coil spring,
a spring shaft (91) for mounting on a pre-assembled drive unit of the administration device, wherein the drive unit includes a spring receiving means and a drive member arranged moveably in the spring receiving means, wherein the spring shaft (91) includes a fixedly mounted flange (91b),
where
an inner end of the spring (92) is at least non-rotatably fitted to the spring shaft (91),
a spring sleeve (93) which at least partially surrounds the peripheral region of the spring (92), wherein the outer end (92a) of the spring (92) is non-rotatably connected to the spring sleeve (93),
a disc-like spring sleeve cover (94) which can be fixedly mounted to the spring sleeve (93) or the spring shaft (91), wherein the plate diameter of the spring sleeve cover (94) is equal to or less than that of the spring sleeve (93),
wherein the spring shaft (91) defines an axis which has a proximal end and a distal end and the flange (91b) and the spring sleeve cover (94) extend radially away from the axis,
wherein the flange (91b) is arranged in the proximity of the distal end of the spring shaft (91),
**characterised in that**
arranged at the periphery of the flange (91b) is at least one radial abutment (91c), and
arranged at the spring sleeve (93) in complementary relationship with the at least one radial abutment (91c) is at least one blocking means in the form of a locking snap member (93a) so that upon engagement of the locking snap member (93a) and the radial abutment (91c) the spring sleeve (93) and the spring shaft (91) are releasably non-rotationally secured relative to each other,
wherein the spring (92) is prestressed in the spring assembly (9) so that a torque is exerted on the spring shaft (91) or the spring sleeve (93),
wherein a spring stressing of the prestressed spring (92) can be held by the non-rotational securing action,
wherein the non-rotational securing action can be released by a movement of the locking snap member (93a) so that in a released position a relative rotation is possible between the spring shaft (91) and the spring sleeve (93) so that the torque can be passed from the spring (92) to outside the spring assembly (9).

2. A spring assembly (9) according to claim 1 wherein the axial positioning of the spring (92) is defined by the fixed position of the flange (91) and a variable axial fixing of the spring sleeve cover (94) and thereby springs of different axial extents can be fitted in the spring assembly (9).

3. A spring assembly (9) according to claim 2 wherein the spring sleeve cover (94) can be pushed into the spring sleeve (93), wherein the spring sleeve cover (94) has at least one snap element at its periphery and the spring sleeve (93) has in complementary relationship therewith at least one receiving means for the at least one snap element in order to fix the spring sleeve cover (94) to the spring sleeve (93).

4. A spring assembly (9) according to claim 3 wherein the spring sleeve (93) includes at a plurality of axial positions at least one receiving means for the at least one snap element of the spring sleeve cover (94) so that the spring sleeve cover (94) can be fixed to the spring sleeve (93) in various axial positions.

5. A spring assembly (9) according to one of claims 1 to 4 **characterised in that** the spring (92) comprises spirally wound band material, preferably metallic band material, particularly preferably steel in band form.

6. A spring assembly (9) according to one of the preceding claims wherein the spring sleeve (93) is arranged coaxially relative to the spring shaft (91), **characterised in that** arranged at the spring sleeve (93) is at least one flexible arm which extends in the peripheral direction of the spring sleeve (93) and which is mounted with one end fixedly to the spring sleeve (93) and can be deflected in the radial direction at the other end and wherein arranged at the free end is the blocking means, in particular in the form of a tooth (93g), which by deflection of the at least one flexible arm can be brought into engagement with the radial abutment (91c) or can be released from the engagement so that the flexible arm together with the blocking means can form a locking snap member (93a).

7. A spring assembly (9) according to one of the preceding claims wherein the spring shaft (91) has an axially provided holding rib in which the inner end (92c) of the spring (91), which is in the form of a holding lug, can be non-rotatably anchored, and wherein the spring sleeve (93) has an axially oriented holding structure, at which the outer end (92c) of the spring (2), that is in the form of the holding lug, can be non-rotatably anchored.

8. A spring assembly (9) according to claim 6 and claim 7 wherein the spring (92) can be prestressed by relative rotation of the spring sleeve (93) with respect to the spring shaft (91) and wherein said prestressing can be held by engagement of the radial abutment (91c) and the blocking means and wherein the prestressing can correspond to a torque of 1 to 100 Nmm, preferably a torque of 30 to 80 Nmm and particularly preferably a torque of 60 to 70 Nmm.

9. A spring assembly (9) according to claims 6 or 8 wherein a control arm (93b) is arranged at the free end of the arm displaced axially in the distal direction relative to the blocking means and wherein upon radial deflection of the control arm (93b) the blocking means is also correspondingly radially moved and vice-versa.

10. An administration device for the administration of a fluid product, the administration device including
a longitudinal axis,
a housing (2) having a mechanism holder (5) fixedly connected to the housing (2),
a release device,
a product container (13), in particular in the form of a prefilled syringe or carpule which is arranged at least axially fixedly in a part of the housing (2), wherein arranged in the product container (13) is an axially displaceable plug, by the displacement of which in the distal direction product can be distributed from the product container (13),
a spring assembly (9) according to claim 9 in which energy for automatic distribution of product is stored, wherein the spring assembly (9) is operatively connected to the release device and wherein the spring sleeve (93) is non-rotatably connected to the housing (2),
a threaded rod (11) which is arranged coaxially with respect to the longitudinal axis and which is non-rotatably connected to the spring shaft (91),
a piston rod (7) which is arranged coaxially relative to the longitudinal axis and which is guided axially displaceably and non-rotatably in the mechanism holder (5), wherein the piston rod (7) can interact with the plug of the product container (13) in such a way that upon axial movement of the piston rod (7) in the distal direction the plug can also be displaced in the distal direction,
wherein the piston rod (7) is of a sleeve-like configuration and is in threaded engagement with the threaded rod (11) by way of thread elements on an inner surface of the piston road (7) so that rotation of the threaded rod (11) results in axial displacement of the piston rod (7).

11. An administration device according to claim 10 wherein the mechanism holder (5) is connected to a housing portion (12) by a bayonet connection by guide elements of the mechanism holder (5) being in the form of bayonet projections (5h) and a guide along the periphery of the housing portion (12) being in the form of bayonet grooves (12c) so that the bayonet projections (5h) can engage into the bayonet grooves (12c).

12. An administration device according to claim 10 or claim 11 wherein the administration device is an autoinjector (0) or an injection pen with automatic administration.

13. An administration device according to claim 10 or claim 11 wherein the administration device is a "patch" device.

14. A method of installing a spring assembly (9) having a prestressed drive or coil spring (92) in an administration device for the administration of a fluid product, in particular an elongate injection device, comprising a housing (2) and a mechanism holder (5) fixed with respect to the housing, including at least the following steps:
a) the spring assembly (9) is pushed axially on to a pre-assembled drive unit of the administration device, that includes the mechanism holder (5),
whereby a rotatable drive member provided in the drive unit is non-rotatably connected to a spring shaft (91) of the spring assembly (9), and
whereby a release element arranged fixedly on the mechanism holder (5) advances a control arm (93b) of the spring assembly (9),
b) a housing portion (12) or closure portion of the administration device is pushed in the distal direction axially over the spring assembly (9),
whereby the outer peripheral surface of the spring assembly (9) is non-rotatably connected to the housing portion (12) or closure portion,
c) the housing portion (12) or closure portion is rotated relative to the spring assembly (9) and pre-assembled drive unit,
whereby the guide elements of the mechanism holder (5) are guided in guides of the housing portion (12) or closure portion, wherein the guide extends along the periphery of the housing portion (12) or closure portion,
wherein by the rotation the release element moves the control arm (93b) outwardly in the radial direction and thus enables a torque present in the spring assembly (9), whereby a further relative rotation between the housing portion or the closure portion and the mechanism holder (5) occurs until radial block of the spring assembly (9) in engagement with a radial abutment of the mechanism holder (5) and thus a force-locking connection is produced between the spring assembly (9) and the mechanism holder (5).

## Revendications

1. Bloc-ressort (9) pour un dispositif d'administration, comprenant
un ressort (92), lequel est réalisé comme un ressort d'entraînement ou spiral,
une tige de ressort (91) à monter sur une unité d'entraînement préalablement montée du dispositif d'administration,
dans lequel l'unité d'entraînement comprend un logement de ressort et un organe d'entraînement disposé de manière mobile dans le logement de ressort, dans lequel la tige de ressort (91) comprend une bride (91b) appliquée de manière fixe,
dans lequel une extrémité intérieure du ressort (92) est appliquée au moins de manière solidaire en rotation sur la tige de ressort (91),
une douille de ressort (93), laquelle entoure au moins en partie la zone d'enveloppe du ressort (92), dans lequel l'extrémité extérieure (92a) du ressort (92) est reliée de manière solidaire en rotation à la douille de ressort (93),
un couvercle de douille de ressort (94) du type disque, lequel peut être appliqué de manière fixe sur la douille de ressort (93) ou la tige de ressort (91), dans lequel le diamètre du disque du couvercle de douille de ressort (94) est inférieur ou égal à celui de la douille de ressort (93),
dans lequel la tige de ressort (91) définit un axe, présente une extrémité proximale et une extrémité distale et la bride (91b) et le couvercle de douille de ressort (94) s'étendent radialement à distance de l'axe,
dans lequel la bride (91b) est disposée à proximité de l'extrémité distale de la tige de ressort (91)
**caractérisé en ce que**
au moins une butée radiale (91c) est disposée à la périphérie de la bride (91b), et
au moins un moyen de blocage réalisé comme un cliquet de verrouillage (93a) est disposé sur la douille de ressort (93) de manière complémentaire à l'au moins une butée radiale (91c), de sorte que, lors d'un engagement du cliquet de verrouillage (93a) et de la butée radiale (91c), la douille de ressort (93) et la tige de ressort (91) sont bloquées en rotation l'une par rapport à l'autre et de manière libérable,
dans lequel le ressort (92) est précontraint dans le bloc-ressort (9), de sorte qu'un couple est exercé sur la tige de ressort (91) ou la douille de ressort (93),
dans lequel une tension de ressort du ressort (92) précontraint peut être maintenue par le blocage en rotation,
dans lequel le blocage en rotation peut être libéré par un mouvement du cliquet de verrouillage (93a), de sorte que, dans une position déclenchée, une rotation relative entre la tige de ressort (91) et la douille de ressort (93) est possible, de sorte que le couple peut être guidé à partir du ressort (92) vers l'extérieur du bloc-ressort (9).

2. Bloc-ressort (9) selon la revendication 1, dans lequel le positionnement axial du ressort (92) est défini par la position fixe de la bride (91b), ainsi qu'une fixation axiale variable du couvercle de douille de ressort (94), et de ce fait des ressorts avec une étendue axiale différente peuvent être insérés dans le bloc-ressort (9).

3. Bloc-ressort (9) selon la revendication 2, dans lequel le couvercle de douille de ressort (94) peut être déplacé à l'intérieur de la douille de ressort (93), dans lequel le couvercle de douille de ressort (94) présente à sa périphérie au moins un élément d'encliquetage, et la douille de ressort (93) présente de manière complémentaire à cela au moins un logement pour le au moins un élément d'encliquetage, afin de fixer le couvercle de douille de ressort (94) à la douille de ressort (93).

4. Bloc-ressort (9) selon la revendication 3, dans lequel la douille de ressort (93) comprend sur plusieurs positions axiales au moins un logement pour le au moins un élément d'encliquetage du couvercle de douille de ressort (94), de sorte que le couvercle de douille de ressort (94) peut être fixé à la douille de ressort (93) dans différentes positions axiales.

5. Bloc-ressort (9) selon l'une quelconque des revendications 1 à 4, **caractérisé en ce que** le ressort (92) est constitué de matériau en bande enroulé en spirale, de préférence de matériau en bande métallique, de manière particulièrement préférée d'acier en forme de bande.

6. Bloc-ressort (9) selon l'une quelconque des revendications précédentes, dans lequel la douille de ressort (93) est disposée de manière coaxiale par rapport à la tige de ressort (91), **caractérisé en ce qu'**au moins un bras flexible, lequel s'étend dans la direction périphérique de la douille de ressort (93), lequel, avec une extrémité, est appliqué de manière fixe sur la douille de ressort (93) et, à l'autre extrémité, peut être dévié dans la direction radiale, est disposé sur la douille de ressort (93), et dans lequel le moyen de blocage, en particulier sous la forme d'une dent (93g), lequel, par déviation du au moins un bras flexible, peut être amené en engagement avec la butée radiale (91c) ou peut être dégagé de l'engagement, de sorte que le bras flexible peut former un cliquet de verrouillage (93a) conjointement avec le moyen de blocage, est disposé à l'extrémité libre.

7. Bloc-ressort (9) selon l'une quelconque des revendications précédentes, dans lequel la tige de ressort (91) présente une nervure de retenue réalisée de manière axiale, dans laquelle l'extrémité intérieure (92c) du ressort (92) réalisée comme une languette de retenue peut être ancrée de manière solidaire en rotation, et dans lequel la douille de ressort (93) présente une structure de retenue orientée axialement, sur laquelle l'extrémité extérieure (92c) du ressort (92) réalisée comme une languette de retenue peut être ancrée de manière solidaire en rotation.

8. Bloc-ressort (9) selon la revendication 6 et 7, dans lequel le ressort (92) peut être précontraint par rotation relative de la douille de ressort (93) par rapport à la tige de ressort (91), et dans lequel cette précontrainte peut être retenue par un engagement de la butée radiale (91c) et du moyen de blocage, et dans lequel la précontrainte peut correspondre à un couple de 1 à 100 Nmm, peut correspondre de préférence à un couple de 30 à 80 Nmm et peut correspondre de manière particulièrement préférée à un couple de 60 à 70 Nmm.

9. Bloc-ressort (9) selon les revendications 6 ou 8, dans lequel, à l'extrémité libre du bras, un bras de commande (93b) est disposé de manière décalée axialement dans la direction distale par rapport au moyen de blocage, et dans lequel, lors de la déviation radiale du bras de commande (93b), le moyen de blocage est également déplacé radialement de manière correspondante et inversement.

10. Dispositif d'administration pour administrer un produit fluide, le dispositif d'administration comprenant
un axe longitudinal,
un boîtier (2) avec un support de mécanisme (5), lequel est relié de manière fixe au boîtier (2),
un dispositif de déclenchement,
un contenant de produit (13), en particulier sous la forme d'une seringue ou carpule préremplie, lequel est disposé au moins axialement de manière fixe dans une partie du boîtier (2), dans lequel un bouchon mobile axialement, par le déplacement duquel, dans la direction axiale, le produit peut être versé à partir du contenant de produit (13), est disposé dans le contenant de produit (13),
un bloc-ressort (9) selon la revendication 9, dans lequel est stockée de l'énergie pour le versement automatique de produit, dans lequel le bloc-ressort (9) est relié de manière opérationnelle au dispositif de déclenchement, et dans lequel la douille de ressort (93) est reliée de manière solidaire en rotation au boîtier (2),
une tige filetée (11) disposée de manière coaxiale par rapport à l'axe longitudinal, laquelle est reliée de manière solidaire en rotation à la tige de ressort (91),
une tige de piston (7) disposée de manière coaxiale par rapport à l'axe longitudinal, laquelle est mobile axialement et guidée de manière solidaire en rotation dans le support de mécanisme (5), dans lequel la tige de piston (7) peut interagir avec le bouchon du contenant de produit (13) de sorte que, lors d'un mouvement axial de la tige de piston (7) dans la direction distale, le bouchon peut également être déplacé dans la direction distale,
dans lequel la tige de piston (7) est conçue à la façon d'une douille et est en engagement fileté avec la tige filetée (11) par l'intermédiaire d'éléments filetés sur une surface intérieure de la tige de piston (7), de sorte qu'une rotation de la tige filetée (11) entraîne un déplacement axial de la tige de piston (7).

11. Dispositif d'administration selon la revendication 10, dans lequel le support de mécanisme (5) est relié à une partie de boîtier (12) par une fermeture à baïonnette, du fait que des éléments de guidage du support de mécanisme (5) sont réalisés comme des cames de baïonnette (5h) et un guidage est effectué le long de la périphérie de la partie de boîtier (12) en tant que rainures de baïonnette (12c), de sorte que les cames de baïonnette (5h) peuvent s'insérer dans les rainures de baïonnette (12c).

12. Dispositif d'administration selon la revendication 10 ou 11, dans lequel, pour le dispositif d'administration, il s'agit d'un auto-injecteur (0) ou d'un stylo injecteur avec administration automatique.

13. Dispositif d'administration selon la revendication 10 ou 11, dans lequel, pour le dispositif d'administration, il s'agit d'un appareil « patch ».

14. Procédé pour le montage d'un bloc-ressort (9) avec un ressort d'entraînement ou spiral (92) précontraint dans un dispositif d'administration pour administrer un produit fluide, en particulier un dispositif d'injection allongé, avec un boîtier (2) et un support de mécanisme (5) fixé au boîtier, comprenant au moins les étapes suivantes :
a) le bloc-ressort (9) est glissé axialement sur une unité d'entraînement préalablement montée du dispositif d'administration, laquelle comprend le support de mécanisme (5), ce qui a pour effet qu'un organe d'entraînement rotatif présent dans l'unité d'entraînement est relié de manière bloquée en rotation à une tige de ressort (91) du bloc-ressort (9), et
ce qui a pour effet qu'un élément de libération disposé de manière fixe sur le support de mécanisme (5) se glisse devant un bras de commande (93b) du bloc-ressort (9),
b) une partie de boîtier (12) ou partie terminale du dispositif d'administration est glissée axialement dans la direction distale par l'intermédiaire du bloc-ressort (9),
ce qui a pour effet que la surface d'enveloppe extérieure du bloc-ressort (9) est reliée de manière bloquée en rotation à la partie de boîtier (12) ou partie terminale,
c) la partie de boîtier (12) ou partie terminale est amenée en rotation par rapport au bloc-ressort (9) et à l'unité d'entraînement préalablement montée,
ce qui a pour effet que les éléments de guidage du support de mécanisme (5) sont guidés dans des guidages de la partie de boîtier (12) ou partie terminale, dans lequel le guidage s'étend le long de la périphérie de la partie de boîtier (12) ou partie terminale, dans lequel, par la rotation, l'élément de libération déplace le bras de commande (93b) dans la direction radiale vers l'extérieur et libère par conséquent un couple présent dans le bloc-ressort (9), ce qui a pour effet qu'une autre rotation relative entre la partie de boîtier ou terminale et le support de mécanisme (5) est évoquée jusqu'à ce qu'un bloc radial du bloc-ressort (9) entre en engagement avec une butée radiale du support de mécanisme (5) et qu'une liaison par coopération de formes entre le bloc-ressort (9) et le support de mécanisme (5) est ainsi produite.
